# EUROPEAN PATENT SPECIFICATION

(11) **EP 2 992 088 B1**
(45) Date of publication and mention of the grant of the patent: **21.08.2019**
(21) Application number: 14780560.0
(22) Date of filing: 30.04.2014
(51) Int. Cl.: A61K 35/545, C12N 5/074, A61K 49/00, G01N 33/50

(54) **CELL THERAPY FOR MYELODYSPLASTIC SYNDROMES**
ZELLTHERAPHIE FÜR MYELODYSPLASTISCHE SYNDROME
TRAITEMENT CELLULAIRE DES SYNDROMES MYÉLODYSPLASIQUES

(30) Priority: 30.04.2013 US 201361817625 P
(43) Date of publication of application: 09.03.2016
(73) Proprietor: Katholieke Universiteit Leuven, 3000 Leuven (BE)
(72) Inventor: ROOBROUCK, Valerie, B-3001 Heverlee (BE); DELFORGE, Michel, B-3000 Leuven (BE); VERFAILLIE, Catherine, M., B-3000 Leuven (BE)
(74) Representative: Cornish, Kristina Victoria Joy
(86) International application number: PCT/IB2014/001666
(87) International publication number: WO 2014/184666

(56) References cited:
- WO-A1-2006/121428
- WO-A2-2006/121454
- US-A1- 2005 181 502
- US-A1- 2006 263 337
- J. L. READING ET AL: "Clinical-Grade Multipotent Adult Progenitor Cells Durably Control Pathogenic T Cell Responses in Human Models of Transplantation and Autoimmunity", THE JOURNAL OF IMMUNOLOGY, 1 April 2013 (2013-04-01), XP55150627, ISSN: 0022-1767, DOI: 10.4049/jimmunol.1202710
- SILVIA MORA-LEE ET AL: "Therapeutic Effects of hMAPC and hMSC Transplantation after Stroke in Mice", PLOS ONE, vol. 7, no. 8, 1 August 2012 (2012-08-01), page e43683, XP55150626, DOI: 10.1371/journal.pone.0043683
- SANDRA A JACOBS ET AL: "Immunological characteristics of human mesenchymal stem cells and multipotent adult progenitor cells", IMMUNOLOGY AND CELL BIOLOGY, vol. 91, no. 1, 1 January 2013 (2013-01-01), pages 32-39, XP055150679, ISSN: 0818-9641, DOI: 10.1038/icb.2012.64

## Description

### FIELD OF THE INVENTION

The disclosure concerns methods for treating myelodysplastic syndromes (MDS). The disclosure is generally directed to administering cells that provide at least one positive clinical effect, i.e., alleviating one or more overt symptoms or underlying biological effects of the disease. The disclosure also relates to drug discovery methods to screen for agents that modulate the ability of the administered cells to achieve these effects. Also disclosed herein are cell banks that can be used to provide cells for administration to a subject, the banks comprising cells having a desired potency for achieving these effects. Also disclosed herein are compositions comprising cells of specific potency for achieving these effects, such as in pharmaceutical compositions. Also disclosed herein are methods for evaluating the dose efficacy of the cells to achieve these effects in a patient by assessing the *in vivo* or *in vitro* effects. Also disclosed herein are diagnostic methods conducted prior to administering the cells to a subject to be treated, including assays to assess the desired potency of the cells to be administered. Also disclosed herein are post-administration diagnostic assays to assess the effect of the cells on a subject being treated and adjust the dosage regimen. These assays can be performed on an ongoing basis along with treatment. The cells described herein are non-embryonic stem, non-germ cells that can be characterized by one or more of the following: extended replication in culture and express markers of extended replication, such as telomerase, express markers of pluripotentiality, and have broad differentiation potential, are not tumorigenic or transformed, and have a normal karyotype.

### BACKGROUND OF THE INVENTION

Primary myelodysplastic syndromes (MDS) are clonal hematopoietic stem cell (HSC) disorders characterized by ineffective hematopoiesis and peripheral cytopenias. Intrinsic defects in the HSC as well as extrinsic defects in the bone marrow (BM) niche all contribute to the MDS pathogenesis. In some patients, immunomodulatory drugs have shown a significant improvement in cytopenias.

Stem cell transplantation, particularly in younger patients (i.e. less than 40 years of age), more severely affected patients, offers the potential for curative therapy. Success of bone marrow transplantation has been found to correlate with severity of MDS as determined by the IPSS score, with patients having a more favorable IPSS score tending to have a more favorable outcome with transplantation (Oosterveld, M., et al. Br J. Haematol 123(1):81-9 (2003)).

### SUMMARY OF THE INVENTION

The inventors have found that certain cells have an ameliorating effect in myelodysplastic syndromes. They have also found that these cells can be used *in vitro* to affect myeloid cell function.

Because the effects can be easily measured, e.g., by observing the effect on total bone marrow cells or peripheral blood cells derived from treated MDS patients, also described herein is a real-time diagnostic marker to assess the efficacy of and adjust the dosage regimen of the cells.

Because *in vitro* and *in vivo* assays exist to measure the ability of the cells to treat myelodysplastic syndromes and produce the desired effects, potent cells can be identified and banked for future off-the-shelf use.

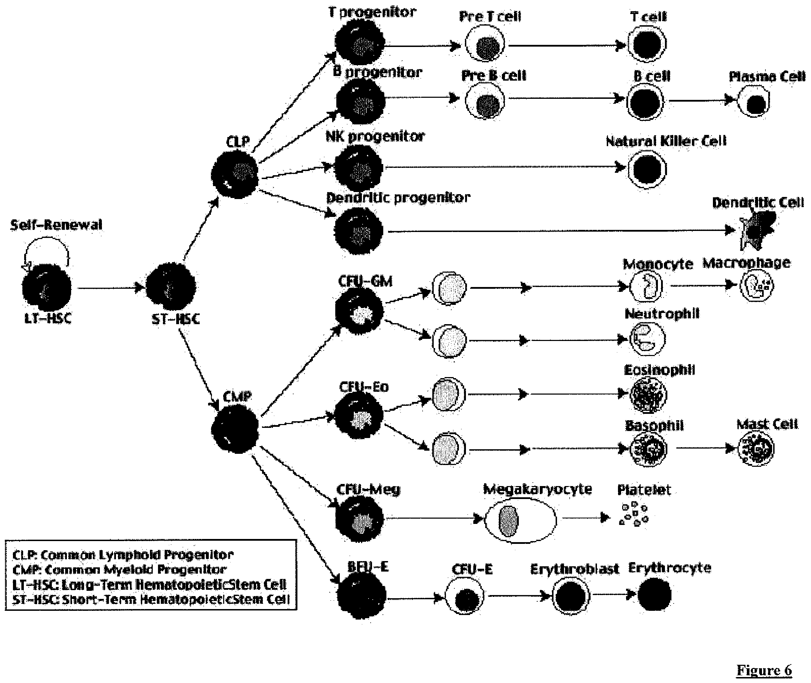

The myelodysplastic syndromes are hematological (blood related) medical conditions manifested with ineffective production (or dysplasia) of the myeloid class of blood cells. Accordingly, patients with MDS can develop severe anemia and require blood transfusions. In some cases, subjects can develop cytopenia (low blood counts) caused by progressive bone marrow failure. Since the myelodysplastic syndromes are all disorders of hematopoietic precursor cells in the bone marrow (e.g., see Figure 6) (only related to myeloid lineage), according to the present invention, treatment with the cells described herein can affect the number and quality of blood-forming cells, reducing their decline and promoting blood production.

Thus, described herein are methods generally for improving the symptoms of MDS, reducing anemia, reducing cytopenia, reducing progressive bone marrow failure, and increasing the number of blood-forming cells in the course of the disease (or preventing or reducing a decline).

In myelodysplastic syndromes, instead of producing healthy mature red blood cells, white blood cells, and platelets, the marrow produces cells that tend to remain immature and to die early. In most subjects with these syndromes, there is a greater number of cells in the marrow compared to healthy subjects (hypercellular marrow), but the cells may not live long enough to exit the marrow into the bloodstream. Or, if they do exit the marrow, they do not remain in circulation for very long before they die. As a result of this, subjects with MDS have low levels of one or more types of blood cell in their bloodstream (cytopenia). Low levels of red blood cells are referred to as anemia, low levels of white blood cells as leucopenia, and low levels of platelets as thrombocytopenia. It is the low levels of these blood cells or low blood counts that cause the overt symptoms of MDS.

These syndromes may be characterized by cells that tend to remain immature. In such patients progression of the disease and efficacy of treatment can be detected not only by the number of mature red cells, white cells, and platelets, but by the number of immature cells, i.e., blast cells, in the bone marrow and blood. This applies to high-risk MDS patients where the percentage of blasts is increased. Low-risk MDS patients have a normal percentage of blasts.

Accordingly, after administration of the cells described herein, cells in the bone marrow and/or peripheral blood can be assessed, including all of the terminally-differentiated cells that include platelets, white blood cells, and red blood cells, as well as, immature precursors of these cells. The dosage may then be adjusted accordingly.

Accordingly, the present disclosure is directed to achieving certain effects, which include the overt physical symptoms of MDS described above and the underlying biological endpoints, such as, abnormal numbers of red cells, white cells, and/or platelets, as well as abnormal numbers of precursors to these terminally-differentiated hematopoietic cells. The cells, therefore, move the numbers of these cells towards more normal levels. That is, they reduce the loss of these terminally differentiated hematopoietic cells and reduce the increase of the more immature (blast) precursor cells.

The above methods are carried out by administering certain cells to a subject. Cells include, but are not limited to, cells that are not embryonic stem cells and not germ cells, having some characteristics of embryonic stem cells, but being derived from non-embryonic tissue, and providing the effects described in this application. The cells may naturally achieve these effects (i.e., not genetically or pharmaceutically modified). However, natural expressors can be genetically or pharmaceutically modified to increase potency.

The cells may express pluripotency markers, such as oct4. They may also express markers associated with extended replicative capacity, such as telomerase. Other characteristics of pluripotency can include the ability to differentiate into cell types of more than one germ layer, such as two or three of ectodermal, endodermal, and mesodermal embryonic germ layers. Such cells may or may not be immortalized or transformed in culture. The cells may be highly expanded without being transformed and also maintain a normal karyotype. For example, the non-embryonic stem, non-germ cells may have undergone at least 10-40 cell doublings in culture, such as 50, 60, or more, wherein the cells are not transformed and have a normal karyotype. The cells may differentiate into at least one cell type of each of two of the endodermal, ectodermal, and mesodermal embryonic lineages and may include differentiation into all three. Further, the cells may not be tumorigenic, such as not producing teratomas. If cells are transformed or tumorigenic, and it is desirable to use them for infusion, such cells may be disabled so they cannot form tumors *in vivo,* as by treatment that prevents cell proliferation into tumors. Such treatments are well known in the art.

Cells described herein include:
1. Isolated expanded non-embryonic stem, non-germ cells, the cells having undergone at least 10-40 cell doublings in culture, wherein the cells express oct4, are not transformed, and have a normal karyotype.
2. The non-embryonic stem, non-germ cells of 1 above that further express one or more of telomerase, rex-1, rox-1, or sox-2.
3. The non-embryonic stem, non-germ cells of 1 above that can differentiate into at least one cell type of at least two of the endodermal, ectodermal, and mesodermal embryonic lineages.
4. The non-embryonic stem, non-germ cells of 3 above that further express one or more of telomerase, rex-1, rox-1, or sox-2.
5. The non-embryonic stem, non-germ cells of 3 above that can differentiate into at least one cell type of each of the endodermal, ectodermal, and mesodermal embryonic lineages.
6. The non-embryonic stem, non-germ cells of 5 above that further express one or more of telomerase, rex-1, rox-1, or sox-2.
7. Isolated expanded non-embryonic stem, non-germ cells that are obtained by culture of non-embryonic, non-germ tissue, the cells having undergone at least 40 cell doublings in culture, wherein the cells are not transformed and have a normal karyotype.
8. The non-embryonic stem, non-germ cells of 7 above that express one or more of oct4, telomerase, rex-1, rox-1, or sox-2.
9. The non-embryonic stem, non-germ cells of 7 above that can differentiate into at least one cell type of at least two of the endodermal, ectodermal, and mesodermal embryonic lineages.
10. The non-embryonic stem, non-germ cells of 9 above that express one or more of oct4, telomerase, rex-1, rox-1, or sox-2.
11. The non-embryonic stem, non-germ cells of 9 above that can differentiate into at least one cell type of each of the endodermal, ectodermal, and mesodermal embryonic lineages.
12. The non-embryonic stem, non-germ cells of 11 above that express one or more of oct4, telomerase, rex-1, rox-1, or sox-2.
13. Isolated expanded non-embryonic stem, non-germ cells, the cells having undergone at least 10-40 cell doublings in culture, wherein the cells express telomerase, are not transformed, and have a normal karyotype.
14. The non-embryonic stem, non-germ cells of 13 above that further express one or more of oct4, rex-1, rox-1, or sox-2.
15. The non-embryonic stem, non-germ cells of 13 above that can differentiate into at least one cell type of at least two of the endodermal, ectodermal, and mesodermal embryonic lineages.
16. The non-embryonic stem, non-germ cells of 15 above that further express one or more of oct4, rex-1, rox-1, or sox-2.
17. The non-embryonic stem, non-germ cells of 15 above that can differentiate into at least one cell type of each of the endodermal, ectodermal, and mesodermal embryonic lineages.
18. The non-embryonic stem, non-germ cells of 17 above that further express one or more of oct4, rex-1, rox-1, or sox-2.
19. Isolated expanded non-embryonic stem, non-germ cells that can differentiate into at least one cell type of at least two of the endodermal, ectodermal, and mesodermal embryonic lineages, said cells having undergone at least 10-40 cell doublings in culture.
20. The non-embryonic stem, non-germ cells of 19 above that express one or more of oct4, telomerase, rex-1, rox-1, or sox-2.
21. The non-embryonic stem, non-germ cells of 19 above that can differentiate into at least one cell type of each of the endodermal, ectodermal, and mesodermal embryonic lineages.
22. The non-embryonic stem, non-germ cells of 21 above that express one or more of oct4, telomerase, rex-1, rox-1, or sox-2.

The subject described herein may be human.

The aspects of the invention for which protection is sought are defined in the claims.

According to a first aspect, the invention provides an *in vitro* method to assess the potency of a cell, the cell being a multipotent adult progenitor cell characterised in that the cell is a non-embryonic, non-germ cell that expresses one or more of oct4, telomerase, rex-1, or rox-1, the method comprising assaying for the potency of the cell to enhance differentiation, proliferation, or lifespan (viability) of red blood cells, leukocytes, platelets, or precursors of these cells.

The non-embryonic, non-germ cells may express telomerase. In this embodiment, the non-embryonic, non-germ cells may be able to differentiate into cell types of at least two of the endodermal, ectodermal, and mesodermal germ layers.

The non-embryonic, non-germ cells may be able to differentiate into cell types of at least two of endodermal, ectodermal, and mesodermal germ layers. The non-embryonic, non-germ cells may be able to differentiate into cell types of all three of the endodermal, ectodermal, and mesodermal germ layers.

The non-embryonic, non-germ cells may express oct4. In this embodiment, the non-embryonic, non-germ cells may be able to differentiate into cell types of at least two of the endodermal, ectodermal, and mesodermal germ layers.

In one embodiment, the non-embryonic, non-germ cells express oct4 and telomerase and can differentiate into cell types of all three of the endodermal, ectodermal, and mesodermal germ layers.

The non-embryonic, non-germ cells may be derived from human bone marrow.

In one embodiment, the non-embryonic, non-germ cells are not tumorigenic or transformed and have a normal karyotype.

The assaying for the potency of the cell may include a colony forming assay. The assaying for the potency of the cell may performed by detecting genes that are modulated by the cells or by detecting genes that are expressed by the cells. The genes may be selected from the group consisting of osteopontin, stem cell factor and Angpt1. The genes that are expressed by the cells may be detected by ELISA, Luminex. qRT-PCR, anti-factor western blots or factor immunohistochemistry.

The method according to the first aspect of the invention may further comprise selecting cells having said potency and optionally further comprising creating a cell bank comprising the cells having said potency.

According to a second aspect, the invention provides cells for use in a method for treating a myelodysplastic syndrome in a subject, the cells being non-embryonic, non-germ cells that express one or more of oct4, telomerase, rex-1, or rox-1, wherein the cells have been assessed for potency according to the method of the first aspect of the invention and wherein the cells have been found to have a potency to enhance differentiation, proliferation, or lifespan (viability) of red blood cells, leukocytes, platelets, or precursors of these cells.

In view of the property of the cells to achieve the desired effects, the cells can be used in drug discovery methods to screen for an agent that affects the ability of the cells to achieve any of the effects. Such agents include, but are not limited to, small organic molecules, antisense nucleic acids, siRNA DNA aptamers, peptides, antibodies, non-antibody proteins, cytokines, chemokines, and chemo-attractants.

In view of the property of the cells to achieve the effects, cell banks can be established containing cells that are selected for having a desired potency to achieve any of the effects. The bank can provide a source for making a pharmaceutical composition to administer to a subject. Cells can be used directly from the bank or expanded prior to use. Especially in the case that the cells are subjected to further expansion, after expansion it is desirable to validate that the cells still have the desired potency. Banks allow the "off the shelf" use of cells that are allogeneic to the subject.

Accordingly, also described herein are diagnostic procedures conducted prior to administering the cells to a subject. The procedures include assessing the potency of the cells to achieve the effects described in this application. The cells may be taken from a cell bank and used directly or expanded prior to administration. In either case, the cells could be assessed for the desired potency. Especially in the case that the cells are subjected to further expansion, after expansion it is desirable to validate that the cells still have the desired potency. Or the cells can be derived from the subject and expanded prior to administration. In this case, as well, the cells could be assessed for the desired potency prior to administration back to the subject (autologous).

In a clinical setting, one may administer the cells after obtaining a baseline by assaying for numbers of the various red and white blood cells and platelets, as well as their immature precursors, either directly or by means of gene expression, and, then, following administration of the cells during treatment, monitor one or more times for one or more of these effects. One could then determine the optimized dose for treatment.

Accordingly, also described herein are diagnostic procedures conducted prior to administering the cells to a subject, the pre-diagnostic procedures including assessing the potency of the cells to achieve one or more of the desired effects. The cells may be taken from a cell bank and used directly or expanded prior to administration. In either case, the cells would be assessed for the desired potency. Or the cells can be derived from the subject and expanded prior to administration. In this case, as well, the cells would be assessed for the desired potency prior to administration.

Although the cells selected for the effects are necessarily assayed during the selection procedure, it may be preferable and prudent to again assay the cells prior to administration to a subject for treatment to confirm that the cells still achieve the effects at desired levels. This is particularly preferable where the cells have been stored for any length of time, such as in a cell bank, where cells are most likely frozen during storage.

With respect to methods of treatment with cells that achieve the desired effects, between the original isolation of the cells and the administration to a subject, there may be multiple (i.e., sequential) assays for the effects. This is to confirm that the cells can still achieve the effects, at desired levels, after manipulations that occur within this time frame. For example, an assay may be performed after each expansion of the cells. If cells are stored in a cell bank, they may be assayed after being released from storage. If they are frozen, they may be assayed after thawing. If the cells from a cell bank are expanded, they may be assayed after expansion. Preferably, a portion of the final cell product (that is physically administered to the subject) may be assayed.

Also described herein are post-treatment diagnostic assays, following administration of the cells, to assess efficacy.

Also described herein are a method for establishing the dosage of such cells by assessing the potency of the cells to achieve one or more of the above effects. In this case, the potency would be determined and the dosage adjusted accordingly

In this case, one would monitor efficacy, by methods including one or more of the assays described in this application, to establish and maintain a proper dosage regimen.

Also described herein are compositions comprising a population of the cells having a desired potency to achieve the desired effects. Such populations may be found as pharmaceutical compositions suitable for administration to a subject and/or in cell banks from which cells can be used directly for administration to a subject or expanded prior to administration. The cells may have enhanced (increased) potency compared to the previous (parent) cell population. Parent cells are as defined herein. Enhancement can be by selection of natural expressors or by external factors acting on the cells.

Accordingly, any of the indicators described herein may be monitored during treatment with the methods and cells according to the current disclosure.

For all these treatments, one would administer the cells that achieve the effects described in this application. Such cells could have been assessed for the potency and selected for desired potency.

It is understood, however, that for treatment of any of the above conditions, it may be expedient to use such cells; that is, one that has been assessed for achieving the desired effects and selected for a desired level of efficacy prior to administration for treatment of the condition.

The pathology may be the result of the failure of normal proliferation and/or differentiation of myeloid precursor cells and the cells are non-embryonic, non-germ cells that express pluripotentiality markers, e.g., one or more of telomerase, rex-1, sox-2, oct4, rox-1, nanog, SSEA-1, and SSEA-4, and/or have broad differentiation potential, e.g., at least two of ectodermal, endodermal, and mesodermal cell types.

The cells may be prepared by the isolation and culture conditions described herein. They may be prepared by culture conditions that are described herein involving
lower oxygen concentrations combined with higher serum, such as those used to prepare the cells designated "MultiStem®."

### BRIEF DESCRIPTION OF THE FIGURES

Figure 1 - The age, BM cellularity and percentage of CD34⁺ cells in young controls (n=16), age-matched controls (n=7), MDS patients (n=17) and patients with unknown cytopenias (n=7).
Figure 2 - CFC assay with and without MultiStem (donor SJA) provided in a transwell above the culture. Shown are the numbers of BFU-E and CFU-GM colonies in patients and age-matched controls.
Figure 3 - CFC assay with and without MAPC (donor B30E2) provided in a transwell above the culture. Shown are the numbers of BFU-E and CFU-GM colonies in patients and age-matched controls.
Figure 4 - Left panel = LTC-IC assay with and without MultiStem (donor SJA) provided in a transwell above the culture. Shown is the number of total LTC-ICs per 15.000 CD34⁺ cells plated in patients and age-matched controls. Right panel = Phase-contrast morphology of CFU-GM colonies from a MDS patient sample with and without MultiStem (50x, Axiovert 40C, Zeiss).
Figure 5 - LTC-IC assay with and without MultiStem (donor SJA en donor SVG) provided in a transwell above the culture, with MultiStem medium alone (in transwell), with MultiStem (SJA) in direct contact, with MultiStem (SJA) given intermittently. Shown is the number of total LTC-ICs per 15.000 CD34⁺ cells plated in patients with low-risk MDS and unknown cytopenias.
Figure 6 - Schematic of hematopoietic development. CLP: Common Lymphoid Progenitor. CMP: Common Myeloid Progenitor. LT-HSC: Long-Term Hematopoietic Stem Cell. ST-HSC: Short-Term Hematopoietic Stem Cell.

### DETAILED DESCRIPTION OF THE INVENTION

The section headings are used herein for organizational purposes only and are not to be construed as in any way limiting the subject matter described.

The methods and techniques of the present application are generally performed according to conventional methods well-known in the art and as described in various general and more specific references that are cited and discussed throughout the present specification unless otherwise indicated. See, e.g., Sambrook et al., Molecular Cloning: A Laboratory Manual, 3rd ed., Cold Spring Harbor Laboratory Press, Cold Spring Harbor, N.Y. (2001) and Ausubel et al., Current Protocols in Molecular Biology, Greene Publishing Associates (1992), and Harlow and Lane, Antibodies: A Laboratory Manual, Cold Spring Harbor Laboratory Press, Cold Spring Harbor, N.Y. (1990).

### Definitions

"A" or "an" means herein one or more than one; at least one. Where the plural form is used herein, it generally includes the singular.

A "cell bank" is industry nomenclature for cells that have been grown and stored for future use. Cells may be stored in aliquots. They can be used directly out of storage or may be expanded after storage. This is a convenience so that there are "off the shelf' cells available for administration. The cells may already be stored in a pharmaceutical ly-acceptable excipient so they may be directly administered or they may be mixed with an appropriate excipient when they are released from storage. Cells may be frozen or otherwise stored in a form to preserve viability. In one embodiment of the invention, cell banks are created in which the cells have been selected for enhanced potency to achieve the effects described in this application. Following release from storage, and prior to administration to the subject, it may be preferable to again assay the cells for potency. This can be done using any of the assays, direct or indirect, described in this application or otherwise known in the art. Then cells having the desired potency can then be administered to the subject for treatment. Banks can be made using cells derived from the individual to be treated (from their pre-natal tissues such as placenta, umbilical cord blood, or umbilical cord matrix or expanded from the individual at any time after birth). Or banks can contain cells for allogeneic uses.

"Co-administer" means to administer in conjunction with one another, together, coordinately, including simultaneous or sequential administration of two or more agents.

"Comprising" means, without other limitation, including the referent, necessarily, without any qualification or exclusion on what else may be included. For example, "a composition comprising x and y" encompasses any composition that contains x and y, no matter what other components may be present in the composition. Likewise, "a method comprising the step of x" encompasses any method in which x is carried out, whether x is the only step in the method or it is only one of the steps, no matter how many other steps there may be and no matter how simple or complex x is in comparison to them. "Comprised of and similar phrases using words of the root "comprise" are used herein as synonyms of "comprising" and have the same meaning.

"Comprised of" is a synonym of "comprising" (see above).

"EC cells" were discovered from analysis of a type of cancer called a teratocarcinoma. In 1964, researchers noted that a single cell in teratocarcinomas could be isolated and remain undifferentiated in culture. This type of stem cell became known as an embryonic carcinoma cell (EC cell).

"Effective amount" generally means an amount which provides the desired local or systemic effect, e.g., effective to ameliorate undesirable effects of MDS, including achieving the specific desired effects described in this application. For example, an effective amount is an amount sufficient to effectuate a beneficial or desired clinical result. The effective amounts can be provided all at once in a single administration or in fractional amounts that provide the effective amount in several administrations. The precise determination of what would be considered an effective amount may be based on factors individual to each subject, including their size, age, injury, and/or disease or injury being treated, and amount of time since the injury occurred or the disease began. One skilled in the art will be able to determine the effective amount for a given subject based on these considerations which are routine in the art. As used herein, "effective dose" means the same as "effective amount."

"Effective route" generally means a route which provides for delivery of an agent to a desired compartment, system, or location. For example, an effective route is one through which an agent can be administered to provide at the desired site of action an amount of the agent sufficient to effectuate a beneficial or desired clinical result.

"Embryonic Stem Cells (ESC)" are well known in the art and have been prepared from many different mammalian species. ESCs are described herein for reference only as they are not covered by the claims. Embryonic stem cells are stem cells derived from the inner cell mass of an early stage embryo known as a blastocyst. They are able to differentiate into all derivatives of the three primary germ layers: ectoderm, endoderm, and mesoderm. These include each of the more than 220 cell types in the adult body. The ES cells can become any tissue in the body, excluding placenta. Only the morula's cells are totipotent, able to become all tissues and a placenta. Some cells similar to ESCs may be produced by nuclear transfer of a somatic cell nucleus into an enucleated fertilized egg.

Use of the term "includes" is not intended to be limiting.

"Hematopoietic stem cells" are the blood cells that give rise to all the other blood cells and are derived from mesoderm.

They give rise to the myeloid (monocytes and macrophages, neutrophils, basophils, eosinophils, erythrocytes, megakaryocytes/platelets, dendritic cells), and lymphoid lineages (T-cells, B-cells, NK-cells). The definition of hematopoietic stem cells has changed in the last two decades. The hematopoietic tissue contains cells with long-term and short-term regeneration capacities and committed multipotent, oligopotent, and unipotent progenitors. HSCs constitute 1:10.000 of cells in myeloid tissue.

HSCs are a heterogeneous population. Three classes of stem cells exist, distinguished by their ratio of lymphoid to myeloid progeny (L/M) in blood. Myeloid-biased (My-bi) HSC have low L/M ratio (>0, <3), whereas lymphoid-biased (Ly-bi) HSC show a large ratio (>10). The third category consists of the balanced (Bala) HSC for which 3 ≤ L/M ≤ 10. Only the myeloid-biased and - balanced HSCs have durable self-renewal properties. In addition, serial transplantation experiments have shown that each subtype preferentially re-creates its blood cell type distribution, suggesting an inherited epigenetic program for each subtype.

As stem cells, HSC are defined by their ability to replenish all blood cell types and their ability to self-renew.

Stem cell heterogeneity It was originally believed that all HSC were alike in their self-renewal and differentiation abilities. This view was first challenged by the 2002 discovery by the Muller-Sieburg group in San Diego, who illustrated that different stem cells can show distinct repopulation patterns that are epigenetically predetermined intrinsic properties of clonal Thy-1^{lo} SCA-1⁺ lin⁻ c-kit⁺ HSC.^{[3][4][5]} The results of these clonal studies led to the notion of lineage bias. Using the ratio *ρ* = *L*/*M* of lymphoid (L) to myeloid (M) cells in blood as a quantitative marker, the stem cell compartment can be split into three categories of HSC. Balanced (Bala) HSC repopulate peripheral white blood cells in the same ratio of myeloid to lymphoid cells as seen in unmanipulated mice (on average about 15% myeloid and 85% lymphoid cells, or 3≤ρ≤10). Myeloid-biased (My-bi) HSC give rise to too few lymphocytes resulting in ratios 0<ρ<3, while lymphoid-biased (Ly-bi) HSC generate too few myeloid cells, which results in lymphoid-to-myeloid ratios of 10<ρ<oo. All three types are norm three types of HSC, and they do not represent stages of differentiation. Rather, these are three classes of HSC, each with an epigenetically fixed differentiation program. These studies also showed that lineage bias is not stochastically regulated or dependent on differences in environmental influence. My-bi HSC self-renew longer than balanced or Ly-bi HSC. The myeloid bias results from reduced responsiveness to the lymphopoetin Interleukin 7 (IL-7).^{[4]}

Subsequent to this, other groups confirmed and highlighted the original findings^{[6]}. For example, the Eaves group confirmed in 2007 that repopulation kinetics, long-term self-renewal capacity, and My-bi and Ly-bi are stably inherited intrinsic HSC properties.^{[7]} In 2010, the Goodell group provided additional insights about the molecular basis of lineage bias in side population Side population (SP) SCA-1⁺ lin⁻ c-kit⁺ HSC.^{[8]} As previously shown for IL-7 signaling, it was found that a member of the transforming growth factor family (TGF-beta) induces and inhibits the proliferation of My-bi and Ly-bi HSC, respectively.

Markers In reference to phenotype, hematopoeitic stem cells are identified by their small size, lack of lineage (lin) markers, low staining (side population) with vital dyes such as rhodamine 123 (rhodamine^{DULL}, also called rho^{lo}) or Hoechst 33342, and presence of various antigenic markers on their surface.

Cluster of differentiation and other markers Many of these markers belong to the cluster of differentiation series, like: CD34, CD38, CD90, CD133, CD105, CD45, and also c-kit, - the receptor for stem cell factor. The hematopoietic stem cells are negative for the markers that are used for detection of lineage commitment, and are, thus, called Lin-; and, during their purification by FACS, a bunch of up to 14 different mature blood-lineage marker, e.g., CD13 & CD33 for myeloid, CD71 for erythroid, CD19 for B cells, CD61 for megakaryocytic, etc. for humans; and, B220 (murine CD45) for B cells, Mac-1 (CD11b/CD18)) for monocytes, Gr-1 for Granulocytes, Ter119 for erythroid cells, I17Ra, CD3, CD4, CD5, CD8 for T cells, etc. (for mice) antibodies are used as a mixture to deplete the lin+ cells or late multipotent progenitors (MPP)s.

There are many differences between the human and mice hematopoietic cell markers for the commonly accepted type of hematopoietic stem cells^{[t]}.
- Mouse HSC : CD34^{lo/-}, SCA-1⁺, Thy1.1^{+/lo}, CD38⁺, C-kit⁺, lin⁻
- Human HSC : CD34⁺, CD59⁺, Thy1/CD90⁺, CD38^{lo/-}, C-kit/CD117⁺, lin⁻

SLAM code Alternative methods that could give rise to similar or better harvest of stem cells are presently emerging. One such method uses a signature of *SLAM* family of cell surface molecules. SLAM (Signaling lymphocyte activation molecule) family is a group of >10 molecules whose genes are located mostly tandemly in a single locus on chromosome 1 (mouse), all belonging to a subset of immunoglobulin gene superfamily, and originally thought to be involved in T-cell stimulation. This family includes CD48, CD150, CD244, etc., CD150 being the founding member, and, thus, also called slamF1, i.e., SLAM family member 1.

The signature SLAM code for the hemopoietic hierarchy are:
- Hematopoietic stem cells (HSC) : CD150⁺ CD48⁻CD244⁻
- Multipotent progenitor cells (MPPs) : CD150⁻ CD48⁻ CD244⁺
- Lineage-restricted progenitor cells (LRPs) : CD150⁻ CD48⁺ CD244⁺
- Common myeloid progenitor (CMP) : lin SCA-1⁻c-kit⁺CD34⁺CD16/32^{mid}
- Granulocyte-macrophage progenitor (GMP) : lin SCA-1⁻c-kit⁺CD34⁺CD16/32^{hi}
- Megakarocyte-erythroid progenitor (MEP) : lin SCA-1⁻c-kit⁺CD34⁻CD16/32^{low}

For HSCs, CD150⁺CD48⁻ was sufficient instead of CD150⁺CD48⁻CD244⁻ because CD48 is a ligand for CD244, and both would be positive only in the activated lineage-restricted progenitors. Recent work has shown that this method excludes a large number of HSCs and includes an equally large number of non-stem cells.^{[13][14]} CD150⁺CD48⁻ gave stem cell purity comparable to Thy1^{lo}SCA-1⁺lin c-kit⁺ in mice.^{[15]}

Osteoclasts also arise from hemopoietic cells of the monocyte/neutrophil lineage, specifically CFU-GM.

| Committee | "lympho" | "rubri" | "granulo" or "myelo" | "mono" | "megakaryo" |
|---|---|---|---|---|---|
| *Lineage* | Lymphoid | Myeloid | Myeloid | Myeloid | Myeloid |
| *CFU* | CFU-L | CFU-GEMM→CFU-E | CFU-GEMM→CFU-GM→CFU-G | CFU-GEMM→CFU-GM→CFU-M | CFU-GEMM→CFU-Meg |
| *Process* | lymphocytopoiesis | erythropoiesis | granulocytopoiesis | monocytopoiesis | thrombocytopoiesis |
| *[root]blast* | Lymphoblast | Proerythroblast | Myeloblast | Monoblast | Megakaryoblast |
| *pro[root]cyte* | Prolymphocyte | Polychromatophilic erythrocyte | Promyelocyte | Promonocyte | Promegakaryocyte |
| *[root]cyte* | - | Normoblast | Eosino/neutro/basophilic myelocyte | | Megakaryocyte |
| *meta[root]cyte* | Large lymphocyte | Reticulocyte | Eosinophilic/neutrophitic/basophilic metamyelocyte, Eosinophilic/neutrophilic/basophilic band cell | Early monocyte | - |
| *mature cell name* | Small lymphocyte | Erythrocyte | granulocytes (Eosino/neutron/basophil) | Monocyte | Thrombocytes (Platelets) |

### Colony-forming units

There are various kinds of colony-forming units:
- Colony-forming unit lymphocyte (CFU-L)
- Colony-forming unit erythrocyte (CFU-E)
- Colony-forming unit granulo-monocyte (CFU-GM)
- Colony-forming unit megakaryocyte (CFU-Me)
- Colony-forming unit Basophil (CFU-B)
- Colony-forming unit Eosinophil (CFU-Eo)
The above CFUs are based on the lineage. Another CFU, the *colony-forming unit-spleen* (CFU-S) was the basis of an *in vivo* clonal colony formation, which depends on the ability of infused bone marrow cells to give rise to clones of maturing hematopoietic cells in the spleens of irradiated mice after 8 to 12 days. It was used extensively in early studies, but is now considered to measure more mature progenitor or Transit Amplifying Cells rather than stem cells.
1. "5. Hematopoietic Stem Cells." Stem Cell Information. National Institutes of Health, U.S. Department of Health and Human Services, 17 Jun 2011. Web. 9 Nov 2013. <http://stemcells.nih.gov/info/scirepost/pages/chapter5.aspx>
2. Dzierzak & Speck, Of lineage and legacy: the development of mammalian hematopoietic stem cells, Nature Immunology, 2008
3. Muller-Sieburg CE, Cho RH, Thoman M, Adkins B, Sieburg HB, Deterministc regulation of haematopoietic stem cell self-renewal and differentiation. Blood. 2002; 100; 1302-9
4. *^{b}* Muller-Sieburg CE, Cho RH, Karlson L, Huang JF, Sieburg HB. Myeloid-biased hematopoietic stem cells have extensive self-renewal capacity but generate diminished progeny with impaired IL-7 responsiveness. Blood. 2004; 103:4111-8
5. Sieburg HB, Cho RH, Dykstra B, Eaves, CJ, Muller-Sieburg, CE. The haematopoietic stem cell compartment consists of a limited number of discrete stem cell subsets. Blood. 2006; 107:2311-6. Epub 2005 Nov 15
6. Schroeder, T. Haematopoietic Stem Cell Heterogeneity: Subtypes, Not Unpredictable Behavior. Cell Stem Cell 2010. DOI 10.1016/j.stem.2010.02.006
7. Dykstra, B et al. Long-Term Propagation of Distinct Hematopoietic Differentiation Programs In Vivo. Cell Stem Cell, Volume 1, Issue 2, 218-229, 16 August 2007
8. Challen, G., Boles, NC, Chambers, SM, Goodell, MA. Distinct Haematopoietic Stem Cell Subtypes Are Differentially Regulated by TGF-beta1. Cell Stem Cel 2010. DOI 10.1016/j.stem.2010.02.002
13. David C Weksberg, Stuart M Chambers, Nathan C Boles, and Margaret A Goodell CD150 negative Side Population cells represent a functionally distinct population of long-term haematopoietic stem cells. Blood 2007 : blood-2007-09-115006v1
14. Gary Van Zant Stem cell markers: less is more! Blood 107: 855-856.
15. Kiel et al., Cell, Vol. 121, 1109-1121, July 1, 2005, Copyright ©2005 by Elsevier Inc. DOI 10.1016/j.cell.2005.05.026

"Increase" or "increasing" means to induce a biological event entirely or to increase the degree of the event.

"Induced pluripotent stem cells (IPSC or IPS cells)" are somatic cells that have been reprogrammed, for example, by introducing exogenous genes that confer on the somatic cell a less differentiated phenotype. These cells can then be induced to differentiate into less differentiated progeny. IPS cells have been derived using modifications of an approach originally discovered in 2006 (Yamanaka, S. et al., Cell Stem Cell, 1:39-49 (2007)). For example, in one instance, to create IPS cells, scientists started with skin cells that were then modified by a standard laboratory technique using retroviruses to insert genes into the cellular DNA. In one instance, the inserted genes were Oct4, Sox2, Lif4, and c-myc, known to act together as natural regulators to keep cells in an embryonic stem cell-like state. These cells have been described in the literature. See, for example, Wernig et al., PNAS, 105:5856-5861 (2008); Jaenisch et al., Cell, 132:567-582 (2008); Hanna et al., Cell, 133:250-264 (2008); and Brambrink et al., Cell Stem Cell, 2:151-159 (2008). These references teach IPSCs and methods for producing them. It is also possible that such cells can be created by specific culture conditions (exposure to specific agents).

The term "isolated" refers to a cell or cells which are not associated with one or more cells or one or more cellular components that are associated with the cell or cells *in vivo.* An "enriched population" means a relative increase in numbers of a desired cell relative to one or more other cell types *in vivo* or in primary culture.

However, as used herein, the term "isolated" does not indicate the presence of only the cells described herein . Rather, the term "isolated" indicates that the cells described herein are removed from their natural tissue environment and are present at a higher concentration as compared to the normal tissue environment. Accordingly, an "isolated" cell population may further include cell types in addition to the cells described herein and may include additional tissue components. This also can be expressed in terms of cell doublings, for example. A cell may have undergone 10, 20, 30, 40 or more doublings *in vitro* or *ex vivo* so that it is enriched compared to its original numbers *in vivo* or in its original tissue environment (e.g., bone marrow, peripheral blood, placenta, umbilical cord, umbilical cord blood, adipose tissue, etc.).

"MAPC" is an acronym for "multipotent adult progenitor cell." It refers to a cell that is not an embryonic stem cell or germ cell but has some characteristics of these. MAPC can be characterized in a number of alternative descriptions, each of which conferred novelty to the cells when they were discovered. They can, therefore, be characterized by one or more of those descriptions. First, they have extended replicative capacity in culture without being transformed (tumorigenic) and with a normal karyotype. Second, they may give rise to cell progeny of more than one germ layer, such as two or all three germ layers (i.e., endoderm, mesoderm and ectoderm) upon differentiation. Third, although they are not embryonic stem cells or germ cells, they may express markers of these primitive cell types so that MAPCs may express one or more of Oct 3/4 (i.e., Oct 3A), rex-1, and rox-1. They may also express one or more of sox-2 and SSEA-4. Fourth, like a stem cell, they may self-renew, that is, have an extended replication capacity without being transformed. This means that these cells express telomerase (i.e., have telomerase activity). Accordingly, the cell type that was designated "MAPC" may be characterized by alternative basic characteristics that describe the cell via some of its novel properties.

The term "adult" in MAPC is non-restrictive. It refers to a non-embryonic somatic cell. MAPCs are karyotypically normal and do not form teratomas *in vivo.* This acronym was first used in U.S. Patent No. 7,015,037 to describe a pluripotent cell isolated from bone marrow. However, cells with pluripotential markers and/or differentiation potential have been discovered subsequently and, for purposes of this invention, may be equivalent to those cells first designated "MAPC." Essential descriptions of the MAPC type of cell are provided in the Summary of the Invention above.

MAPC represents a more primitive progenitor cell population than MSC (Verfaillie, C.M., Trends Cell Biol 12:502-8 (2002), Jahagirdar, B.N., et al., Exp Hematol, 29:543-56 (2001); Reyes, M. and C.M. Verfaillie, Ann N Y Acad Sci, 938:231-233 (2001); Jiang, Y. et al., Exp Hematol, 30896-904 (2002); and (Jiang, Y. et al., Nature, 418:41-9. (2002)).

The term "MultiStem®" is the trade name for a cell preparation based on the MAPCs of U.S. Patent No. 7,015,037, i.e., a non-embryonic stem, non-germ cell as described above. MultiStem® is prepared according to cell culture methods disclosed in this patent application, particularly, lower oxygen and higher serum. MultiStem® is highly expandable, karyotypically normal, and does not form teratomas *in vivo.* It may differentiate into cell lineages of more than one germ layer and may express one or more of telomerase, oct3/4, rex-1, rox-1, sox-2, and SSEA4.

"Myeloid Precursors" are those stem and progenitor cells that normally differentiate into the mature cells of the myeloid lineage: (monocytes and macrophages, neutrophils, basophils, eosinophils, erythrocytes, megakaryocytes/platelets, dendritic cells). These precursors are also shown in Figure 6. Thus, these precursors can be a target of the *in vitro* assays and *in vivo* assessments described in this application. These precursors include the HSCs that ultimately gives rise to the myeloid blood cells.

"Pharmaceutically-acceptable carrier" is any pharmaceutically-acceptable medium for the cells for use according to the present invention. Such a medium may retain isotonicity, cell metabolism, pH, and the like. It is compatible with administration to a subject *in vivo,* and can be used, therefore, for cell delivery and treatment.

The term "potency" refers to the ability of the cells to achieve the various effects described in this application. Accordingly, potency refers to the effect at various levels, including, but not limited to, reducing symptoms of MDS, including, but not limited to, improvement of the myeloid differentiation capacity leading to higher levels of mature blood cells. Potency may also include simulation of the proliferation/differentiation of myeloid precursor cells, a reduction of apoptosis of myeloid precursor cells and a reduction of inflammation. Mature blood cells include red blood cells, also called erythrocytes, as well as white blood cells, also called leukocytes. However, as is also discussed, the potency can refer to effects on thrombocytes or megakaryocytes, i.e., platelet precursors.

"Primordial embryonic germ cells" (PG or EG cells) can be cultured and stimulated to produce many less differentiated cell types. PG or EG cells are described herein for reference only as they are not covered by the claims.

"Progenitor cells" are cells produced during differentiation of a stem cell that have some, but not all, of the characteristics of their terminally-differentiated progeny. Defined progenitor cells, such as "cardiac progenitor cells," are committed to a lineage, but not to a specific or terminally differentiated cell type. The term "progenitor" as used in the acronym "MAPC" does not limit these cells to a particular lineage. A progenitor cell can form a progeny cell that is more highly differentiated than the progenitor cell.

"Red blood cells", also called erythrocytes, are the most common type of blood cell and the vertebrate organism's principal means of delivering oxygen (O₂) to the body tissues via the blood flow through the circulatory system.^{[1]} They take up oxygen in the lungs or gills and release it into tissues while squeezing through the body's capillaries.

Red blood cells are also known as RBCs, red cells,^{[5]} red blood corpuscles (an archaic term), haematids, erythroid cells or erythrocytes.

### Diseases and diagnostic tools

Blood diseases involving the red blood cells include, but are not limited to:
- Anemias (or anaemias) are diseases characterized by low oxygen transport capacity of the blood, because of low red cell count or some abnormality of the red blood cells or the hemoglobin.
   - Iron deficiency anemia is the most common anemia; it occurs when the dietary intake or absorption of iron is insufficient, and hemoglobin, which contains iron, cannot be formed
   - Aplastic anemia is caused by the inability of the bone marrow to produce blood cells.
   - Pure red cell aplasia is caused by the inability of the bone marrow to produce only red blood cells.
- Hemolysis is the general term for excessive breakdown of red blood cells. It can have several causes and can result in hemolytic anemia.
- Polycythemias (or erythrocytoses) are diseases characterized by a surplus of red blood cells. The increased viscosity of the blood can cause a number of symptoms.
   - In polycythemia vera the increased number of red blood cells results from an abnormality in the bone marrow.
- Several microangiopathic diseases, including disseminated intravascular coagulation and thrombotic microangiopathies, present with pathognomonic (diagnostic) red blood cell fragments called schistocytes. These pathologies generate fibrin strands that sever red blood cells as they try to move past a thrombus.
- Hemolytic transfusion reaction is the destruction of donated red blood cells after a transfusion, mediated by host antibodies, often as a result of a blood type mismatch.

Several blood tests involve red blood cells, including the *RBC count* (the number of red blood cells per volume of blood), the hematocrit (percentage of blood volume occupied by red blood cells), and the erythrocyte sedimentation rate. Many diseases involving red blood cells are diagnosed with a blood film (or peripheral blood smear), where a thin layer of blood is smeared on a microscope slide. The blood type needs to be determined to prepare for a blood transfusion or an organ transplantation.
1. "Blood Cells". (http://www.biosbcc.net/doohan/sample/htm/Blood%20cells.htm).
5. Vinay Kumar, Abul K. Abbas, Nelson Fausto, Richard N. Mitchell (2007). Robbins Basic Pathology (8th ed.). Saunders.
46. An X, Mohandas N (May 2008). "Disorders of red cell membrane". British Journal of Haematology 141 (3): 367-75. doi:10.1111/j.1365-2141.2008.07091.x. PMID 18341630.

The term "reduce" as used herein means to prevent as well as decrease. In the context of treatment, to "reduce" is to either prevent or ameliorate one or more clinical symptoms. A clinical symptom is one (or more) that has or will have, if left untreated, a negative impact on the quality of life (health) of the subject. This also applies to the underlying biological effects such as increased apoptosis, increased pro-inflammatory environment, decreased differentiation and/or decreased proliferation of myeloid cell precursors, the end result of which would be to ameliorate the deleterious clinical symptoms of MDS.

"Selecting" a cell with a desired level of potency (e.g., for modulating activation of macrophages) can mean identifying (as by assay), isolating, and expanding a cell. This could create a population that has a higher potency than the parent cell population from which the cell was isolated. The "parent" cell population refers to the parent cells from which the selected cells divided. "Parent" refers to an actual P1 → F1 relationship (i.e., a progeny cell). So if cell X is isolated from a mixed population of cells X and Y, in which X is an expressor and Y is not, one would not classify a mere isolate of X as having enhanced expression. But, if a progeny cell of X is a higher expressor, one would classify the progeny cell as having enhanced expression.

To select a cell that achieves the desired effect would include both an assay to determine if the cells achieve the desired effect and would also include obtaining those cells. The cell may naturally achieve the desired effect in that the effect is not achieved by an exogenous transgene/DNA. But an effective cell may be improved by being incubated with or exposed to an agent that increases the effect. The cell population from which the effective cell is selected may not be known to have the potency prior to conducting the assay. The cell may not be known to achieve the desired effect prior to conducting the assay. As an effect could depend on gene expression and/or secretion, one could also select on the basis of one or more of the genes that cause the effect.

Selection could be from cells in a tissue. For example, in this case, cells would be isolated from a desired tissue, expanded in culture, selected for achieving the desired effect, and the selected cells further expanded.

Selection could also be from cells ex vivo, such as cells in culture. In this case, one or more of the cells in culture would be assayed for achieving the desired effect and the cells obtained that achieve the desired effect could be further expanded.

Cells could also be selected for enhanced ability to achieve the desired effect. In this case, the cell population from which the enhanced cell is obtained already has the desired effect. Enhanced effect means a higher average amount per cell than in the parent population.

The parent population from which the enhanced cell is selected may be substantially homogeneous (the same cell type). One way to obtain such an enhanced cell from this population is to create single cells or cell pools and assay those cells or cell pools to obtain clones that naturally have the enhanced (greater) effect (as opposed to treating the cells with a modulator that induces or increases the effect) and then expanding those cells that are naturally enhanced.

However, cells may be treated with one or more agents that will induce or increase the effect. Thus, substantially homogeneous populations may be treated to enhance the effect.

If the population is not substantially homogeneous, then, it is preferable that the parental cell population to be treated contains at least 100 of the desired cell type in which enhanced effect is sought, more preferably at least 1,000 of the cells, and still more preferably, at least 10,000 of the cells. Following treatment, this sub-population can be recovered from the heterogeneous population by known cell selection techniques and further expanded if desired.

Thus, desired levels of effect may be those that are higher than the levels in a given preceding population. For example, cells that are put into primary culture from a tissue and expanded and isolated by culture conditions that are not specifically designed to produce the effect may provide a parent population. Such a parent population can be treated to enhance the average effect per cell or screened for a cell or cells within the population that express greater degrees of effect without deliberate treatment. Such cells can be expanded then to provide a population with a higher (desired) expression.

"Self-renewal" of a stem cell refers to the ability to produce replicate daughter stem cells having differentiation potential that is identical to those from which they arose. A similar term used in this context is "proliferation."

"Stem cell" means a cell that can undergo self-renewal (i.e., progeny with the same differentiation potential) and also produce progeny cells that are more restricted in differentiation potential. Within the context of the invention, a stem cell would also encompass a more differentiated cell that has de-differentiated, for example, by introduction of specific transcription factors, or by culture under specific conditions. For reference, outside the context of the invention dedifferentiation may also occur by unclear transfer or by fusion with a more primitive stem cell. See, for example, Wilmut et al., Nature, 385:810-813 (1997); Ying et al., Nature, 416:545-548 (2002); Guan et al., Nature, 440:1199-1203 (2006); Takahashi et al., Cell, 126:663-676 (2006); Okita et al., Nature, 448:313-317 (2007); and Takahashi et al., Cell, 131:861-872 (2007).

Dedifferentiation may also be caused by the administration of certain compounds or exposure to a physical environment *in vitro* or *in vivo* that would cause the dedifferentiation. Stem cells also may be derived from abnormal tissue, such as a teratocarcinoma and some other sources such as embryoid bodies (although these can be considered embryonic stem cells in that they are derived from embryonic tissue, although not directly from the inner cell mass). Stem cells may also be produced by introducing genes associated with stem cell function into a non-stem cell, such as an induced pluripotent stem cell.

"Subject" means a vertebrate, such as a mammal, such as a human. Mammals include, but are not limited to, humans, dogs, cats, horses, cows, and pigs.

The term "therapeutically effective amount" refers to the amount of an agent determined to produce any therapeutic response in a mammal. For example, effective anti-inflammatory therapeutic agents may prolong the survivability of the patient, and/or inhibit overt clinical symptoms. Treatments that are therapeutically effective within the meaning of the term as used herein, include treatments that improve a subject's quality of life even if they do not improve the disease outcome per se. Such therapeutically effective amounts are readily ascertained by one of ordinary skill in the art. Thus, to "treat" means to deliver such an amount. Thus, treating can prevent or ameliorate any pathological symptoms of MDS.

"Treat," "treating," or "treatment" are used broadly in relation to the invention and each such term encompasses, among others, preventing, ameliorating, inhibiting, or curing a deficiency, dysfunction, disease, or other deleterious process, including those that interfere with and/or result from a therapy.

"Validate" means to confirm. In the context of the invention, one confirms that a cell is an expressor with a desired potency. This is so that one can then use that cell (in treatment, banking, drug screening, etc.) with a reasonable expectation of efficacy. Accordingly, to validate means to confirm that the cells, having been originally found to have/established as having the desired activity, in fact, retain that activity. Thus, validation is a verification event in a two-event process involving the original determination and the follow-up determination. The second event is referred to herein as "validation."

The cells for use according to the invention are useful for treating any of the myelodysplastic syndromes. This includes, but is not limited to, refractory anemia, which can be characterized by less than 5% primitive blood cells (myeloblasts) in the bone marrow and pathological abnormalities primarily seen in red cell precursors, refractory anemia with ring sideroblasts, also characterized by less than 5% myeloblasts in the bone marrow, but distinguished by the presence of 15% or greater red cell precursors in the marrow, being abnormal iron-stuffed cells called "ring sideroblasts", refractory anemia with excess blasts characterized by 5-20% myeloblasts in the marrow, refractory anemia with excess blasts in transformation, characterized by 21-30% myeloblasts in the marrow (greater than 30% blasts is defined as acute myeloid leukemia), and chronic myelocytic leukemia (not to be confused with chronic myelogenous leukemia), characterized by less than 20% myeloblasts in the bone marrow and greater than 1x10⁹ per liter monocytes (a type of white blood cell) circulating in the peripheral blood.

In general the cells are effective for alleviating the signs and symptoms including anemia (low red blood cell count or reduces hemoglobin), chronic tiredness, shortness of breath, chilled sensation, and sometimes chest pain; neutropenia (low neutrophil count), which can be associated with increased susceptibility to infection; and thrombocytopenia (low platelet count), associated with increased susceptibility to bleeding and bruising (ecchymosis) as well as subcutaneous hemorrhaging resulting in purpura or petechia.

In certain cases, individuals can be asymptomatic and blood cytopenia or other problems are only identified as part of a routine blood count. These problems include neutropenia, anemia, and thrombocytopenia (low cell counts of white and red blood cells and platelets, respectively), splenomegaly or, rarely, hepatomegaly, abnormal granules in cells, abnormal nuclear shape and size, and/or chromosomal abnormalities, including, chromosomal translocations and abnormal chromosome number.

There is some risk for developing acute myelogenous leukemia, but generally, deaths occur as a result of bleeding or infection.

The features generally used to define a MDS are: blood cytopenias; ineffective hematopoiesis; dyserythropoiesis; dysgranulopoiesis; dysmegakaropoiesis and increased myeloblast.

Dysplasia can affect all three lineages seen in the bone marrow. The best way to diagnose dysplasia is by morphology and special stains (PAS) used on the bone marrow aspirate and peripheral blood smear. Dysplasia in the myeloid series is defined by:
- Granulocytic series
   1. Hypersegmented neutrophils (also seen in Vit B₁₂/Folate deficiency)
   2. Hyposegmented neutrophils (Pseudo-Pelger Huet)
   3. Hypogranular neutrophils or pseudo Chediak Higashi large granules
   4. Auer rods - automatically RAEB II (if blast count <5% in the peripheral blood and <10% in the bone marrow aspirate) also note Auer rods may be seen in mature neutrophils in AML with translocation t(8;21)
   5. Dimorphic granules (basophilic and eosinophilic granules) within eosinophils
- Erythroid series
   1. Binucleated erythroid precursors and karyorrhexis
   2. Erythroid nuclear budding
   3. Erythroid nuclear strings or internuclear bridging (also seen in congenital dyserythropoietic anemias)
   4. Loss of E-cadherin in normoblasts is a sign of aberrancy
   5. PAS (globular in vacuoles or diffuse cytoplasmic staining) within erythroid precursors in the bone marrow aspirate (has no bearing on paraffin fixed bone marrow biopsy). Note: One can see PAS vacuolar positivity in L1 and L2 blasts (AFB classification; the L1 and L2 nomenclature is not used in the WHO classification)
   6. Ringed sideroblasts seen on Prussian blue iron stain (10 or more iron granules encircling 1/3 or more of the nucleus and >15% ringed sideroblasts when counted amongst red cell precursors)
- Megakaryocytic series (can be the most subjective)
   1. Hyposegmented nuclear features in platelet producing megakaryocytes (lack of lobation)
   2. Hypersegmented (osteoclastic appearing) megakaryocytes
   3. Ballooning of the platelets (seen with interference contrast microscopy)

Other stains can help in special cases (PAS and napthol ASD chloroacetate esterase positivity) in eosinophils is a marker of abnormality seen in chronic eosinophilic leukemia and is a sign of aberrancy.

On the bone marrow biopsy high grade dysplasia (RAEB-I and RAEB-II) may show atypical localization of immature precursors (ALIPs) which are islands of immature precursor cells (myeloblasts and promyelcytes) localized to the center of intertrabecular space rather than adjacent to the trabeculae or surrounding arterioles. This morphology can be difficult to recognize from treated leukemia and recovering immature normal marrow elements. Also topographic alteration of the nucleated erythroid cells can be seen in early myelodysplasia (RA and RARS), where normoblasts are seen next to bony trabeculae instead of forming normal interstitially placed erythroid islands.

Myelodysplasia is a diagnosis of exclusion and must be made after proper determination of iron stores, vitamin deficiencies, and nutrient deficiencies are ruled out. Also congenital diseases such as congenital dyserythropoietic anemia (CDA I through IV) has been recognized, Pearson's syndrome (sideroblastic anemia), Jordans anomaly - vacuolization in all cell lines may be seen in Chanarin-Dorfman syndrome, ALA (aminolevulinic acid) enzyme deficiency, and other more esoteric enzyme deficiencies are known to give a pseudomyelodysplastic picture in one of the cell lines, however, all three cell lines are never morphologically dysplastic in these entities with the exception of chloramphenicol, arsenic toxicity and other poisons.

All of these conditions are characterized by abnormalities in the production of one or more of the cellular components of blood (red cells, white cells other than lymphocytes and platelets or their progenitor cells, megakaryocytes).

Indicators of a good prognosis: Younger age; normal or moderately reduced neutrophil or platelet counts; low blast counts in the bone marrow(<20%) and no blasts in the blood; no Auer rods; ringed sideroblasts; normal karyotypes of mixed karyotypes without complex chromosome abnormalities and in vitro marrow culture- non leukemic growth pattern.

Indicators of a poor prognosis: Advanced age; severe neutropenia or thrombocytopenia; high blast count in the bone marrow (20-29%) or blasts in the blood; Auer rods; absence of ringed sideroblasts; abnormal localization or immature granulocyte precursors in bone marrow section all or mostly abnormal karyotypes or complex marrow chromosome abnormalities and in-vitro bone marrow culture-leukemic growth pattern.

Prognosis and karyotype: Good: Normal, -Y, del(5q), del(20q)Intermediate or variable: +8, other single or double anomalies Poor; Complex (>3 chromosomal aberrations); chromosome 7 anomalies.

### Stem Cells

The present invention can be practiced, using stem cells only as defined in the claims, which may be of vertebrate species, such as humans, non-human primates, domestic animals, livestock, and other non-human mammals. Types of stem cells (those which are embryonic stem cells and germ cells fall outside the scope of the claims and are marked "for reference" accordingly) are described below.

### Embryonic Stem Cells (for reference)

The most well studied stem cell is the embryonic stem cell (ESC) as it has unlimited self-renewal and multipotent differentiation potential. These cells are derived from the inner cell mass of the blastocyst or can be derived from the primordial germ cells of a post-implantation embryo (embryonal germ cells or EG cells). ES and EG cells have been derived, first from mouse, and later, from many different animals, and more recently, also from non-human primates and humans. When introduced into mouse blastocysts or blastocysts of other animals, ESCs can contribute to all tissues of the animal. ES and EG cells can be identified by positive staining with antibodies against SSEA1 (mouse) and SSEA4 (human). See, for example, U.S. Patent Nos. 5,453,357; 5,656,479; 5,670,372; 5,843,780; 5,874,301; 5,914,268; 6,110,739 6,190,910; 6,200,806; 6,432,711; 6,436,701, 6,500,668; 6,703,279; 6,875,607; 7,029,913; 7,112,437; 7,145,057; 7,153,684; and 7,294,508, which is teach embryonic stem cells and methods of making and expanding them. Accordingly, ESCs and methods for isolating and expanding them are well-known in the art.

A number of transcription factors and exogenous cytokines have been identified that influence the potency status of embryonic stem cells *in vivo.* The first transcription factor to be described that is involved in stem cell pluripotency is Oct4. Oct4 belongs to the POU (Pit-Oct-Unc) family of transcription factors and is a DNA binding protein that is able to activate the transcription of genes, containing an octameric sequence called "the octamer motif" within the promoter or enhancer region. Oct4 is expressed at the moment of the cleavage stage of the fertilized zygote until the egg cylinder is formed. The function of Oct3/4 is to repress differentiation inducing genes (i.e., FoxaD3, hCG) and to activate genes promoting pluripotency (FGF4, Utf1, Rex1). Sox2, a member of the high mobility group (HMG) box transcription factors, cooperates with Oct4 to activate transcription of genes expressed in the inner cell mass. It is essential that Oct3/4 expression in embryonic stem cells is maintained between certain levels. Overexpression or downregulation of >50% of Oct4 expression level will alter embryonic stem cell fate, with the formation of primitive endoderm/mesoderm or trophectoderm, respectively. *In vivo*, Oct4 deficient embryos develop to the blastocyst stage, but the inner cell mass cells are not pluripotent. Instead they differentiate along the extraembryonic trophoblast lineage. Sa114, a mammalian Spalt transcription factor, is an upstream regulator of Oct4, and is therefore important to maintain appropriate levels of Oct4 during early phases of embryology. When Sa114 levels fall below a certain threshold, trophectodermal cells will expand ectopically into the inner cell mass. Another transcription factor required for pluripotency is Nanog, named after a celtic tribe "Tir Nan Og": the land of the ever young. *In vivo*, Nanog is expressed from the stage of the compacted morula, is subsequently defined to the inner cell mass and is downregulated by the implantation stage. Downregulation of Nanog may be important to avoid an uncontrolled expansion of pluripotent cells and to allow multilineage differentiation during gastrulation. Nanog null embryos, isolated at day 5.5, consist of a disorganized blastocyst, mainly containing extraembryonic endoderm and no discernable epiblast.

### Non-Embryonic Stem Cells

Stem cells have been identified in most tissues. Perhaps the best characterized is the hematopoietic stem cell (HSC). HSCs are mesoderm-derived cells that can be purified using cell surface markers and functional characteristics. They have been isolated from bone marrow, peripheral blood, cord blood, fetal liver, and yolk sac. They initiate hematopoiesis and generate multiple hematopoietic lineages. When transplanted into lethally-irradiated animals, they can repopulate the erythroid neutrophil-macrophage, megakaryocyte, and lymphoid hematopoietic cell pool. They can also be induced to undergo some self-renewal cell division. See, for example, U.S. Patent Nos. 5,635,387; 5,460,964; 5,677,136; 5,750,397; 5,681,599; and 5,716,827. U.S. Patent No. 5,192,553 reports methods for isolating human neonatal or fetal hematopoietic stem or progenitor cells. U.S. Patent No. 5,716,827 reports human hematopoietic cells that are Thy-1⁺ progenitors, and appropriate growth media to regenerate them *in vitro.* U.S. Patent No. 5,635,387 reports a method and device for culturing human hematopoietic cells and their precursors. U.S. Patent No. 6,015,554 describes a method of reconstituting human lymphoid and dendritic cells. Accordingly, HSCs and methods for isolating and expanding them are well-known in the art.

Another stem cell that is well-known in the art is the neural stem cell (NSC). These cells can proliferate *in vivo* and continuously regenerate at least some neuronal cells. When cultured ex *vivo*, neural stem cells can be induced to proliferate as well as differentiate into different types of neurons and glial cells. When transplanted into the brain, neural stem cells can engraft and generate neural and glial cells. See, for example, Gage F.H., Science, 287:1433-1438 (2000), Svendsen S.N. et al, Brain Pathology, 9:499-513 (1999), and Okabe S. et al., Mech Development, 59:89-102 (1996). U.S. Patent No. 5,851,832 reports multipotent neural stem cells obtained from brain tissue. U.S. Patent No. 5,766,948 reports producing neuroblasts from newborn cerebral hemispheres. U.S. Patent Nos. 5,564,183 and 5,849,553 report the use of mammalian neural crest stem cells. U.S. Patent No. 6,040,180 reports *in vitro* generation of differentiated neurons from cultures of mammalian multipotential CNS stem cells. WO 98/50526 and WO 99/01159 report generation and isolation of neuroepithelial stem cells, oligodendrocyte-astrocyte precursors, and lineage-restricted neuronal precursors. U.S. Patent No. 5,968,829 reports neural stem cells obtained from embryonic forebrain. Accordingly, neural stem cells and methods for making and expanding them are well-known in the art.

Another stem cell that has been studied extensively in the art is the mesenchymal stem cell (MSC). MSCs are derived from the embryonal mesoderm and can be isolated from many sources, including adult bone marrow, peripheral blood, fat, placenta, and umbilical blood, among others. MSCs can differentiate into many mesodermal tissues, including muscle, bone, cartilage, fat, and tendon. There is considerable literature on these cells. See, for example, U.S. Patent Nos. 5,486,389; 5,827,735; 5,811,094; 5,736,396; 5,837,539; 5,837,670; and 5,827,740. See also Pittenger, M. et al, Science, 284:143-147 (1999).

Another example of an adult stem cell is adipose-derived adult stem cells (ADSCs) which have been isolated from fat, typically by liposuction followed by release of the ADSCs using collagenase. ADSCs are similar in many ways to MSCs derived from bone marrow, except that it is possible to isolate many more cells from fat. These cells have been reported to differentiate into bone, fat, muscle, cartilage, and neurons. A method of isolation has been described in U.S. 2005/0153442.

Other stem cells that are known in the art include gastrointestinal stem cells, epidermal stem cells, and hepatic stem cells, which have also been termed "oval cells" (Potten, C., et al., Trans R Soc Lond B Biol Sci, 353:821-830 (1998), Watt, F., Trans R Soc Lond B Biol Sci, 353:831 (1997); Alison et al., Hepatology, 29:678-683 (1998).

Other non-embryonic cells reported to be capable of differentiating into cell types of more than one embryonic germ layer include, but are not limited to, cells from umbilical cord blood (see U.S. Publication No. 2002/0164794), placenta (see U.S. Publication No. 2003/0181269, umbilical cord matrix (Mitchell, K.E. et al., Stem Cells, 21:50-60 (2003)), small embryonic-like stem cells (Kucia, M. et al., J Physiol Pharmacol, 57 Suppl 5:5-18 (2006)), amniotic fluid stem cells (Atala, A., J Tissue Regen Med, 1:83-96 (2007)), skin-derived precursors (Toma et al., Nat Cell Biol, 3:778-784 (2001)), and bone marrow (see U.S. Publication Nos. 2003/0059414 and 2006/0147246), each of which teach these cells.

### Strategies of Reprogramming Somatic Cells (for reference)

Several different strategies such as nuclear transplantation, cellular fusion, and culture induced reprogramming have been employed to induce the conversion of differentiated cells into an embryonic state. Nuclear transfer involves the injection of a somatic nucleus into an enucleated oocyte, which, upon transfer into a surrogate mother, can give rise to a clone ("reproductive cloning"), or, upon explantation in culture, can give rise to genetically matched embryonic stem (ES) cells ("somatic cell nuclear transfer," SCNT). Cell fusion of somatic cells with ES cells results in the generation of hybrids that show all features of pluripotent ES cells. Explantation of somatic cells in culture selects for immortal cell lines that may be pluripotent or multipotent. At present, spermatogonial stem cells are the only source of pluripotent cells that can be derived from postnatal animals. Transduction of somatic cells with defined factors can initiate reprogramming to a pluripotent state. These experimental approaches have been extensively reviewed (Hochedlinger and Jaenisch, Nature, 441:1061-1067 (2006) and Yamanaka, S., Cell Stem Cell, 1:39-49 (2007)).

### Nuclear Transfer (for reference)

Nuclear transplantation (NT), also referred to as somatic cell nuclear transfer (SCNT), denotes the introduction of a nucleus from a donor somatic cell into an enucleated ogocyte to generate a cloned animal such as Dolly the sheep (Wilmut et al., Nature, 385:810-813 (1997). The generation of live animals by NT demonstrated that the epigenetic state of somatic cells, including that of terminally differentiated cells, while stable, is not irreversible fixed but can be reprogrammed to an embryonic state that is capable of directing development of a new organism. In addition to providing an exciting experimental approach for elucidating the basic epigenetic mechanisms involved in embryonic development and disease, nuclear cloning technology is of potential interest for patient-specific transplantation medicine.

### Fusion of Somatic Cells and Embryonic Stem Cells (for reference)

Epigenetic reprogramming of somatic nuclei to an undifferentiated state has been demonstrated in murine hybrids produced by fusion of embryonic cells with somatic cells. Hybrids between various somatic cells and embryonic carcinoma cells (Solter, D., Nat Rev Genet, 7:319-327 (2006), embryonic germ (EG), or ES cells (Zwaka and Thomson, Development, 132:227-233 (2005)) share many features with the parental embryonic cells, indicating that the pluripotent phenotype is dominant in such fusion products. As with mouse (Tada et al., Curr Biol, 11:1553-1558 (2001)), human ES cells have the potential to reprogram somatic nuclei after fusion (Cowan et al., Science, 309:1369-1373(2005)); Yu et al., Science, 318:1917-1920 (2006)). Activation of silent pluripotency markers such as Oct4 or reactivation of the inactive somatic X chromosome provided molecular evidence for reprogramming of the somatic genome in the hybrid cells. It has been suggested that DNA replication is essential for the activation of pluripotency markers, which is first observed 2 days after fusion (Do and Scholer, Stem Cells, 22:941-949 (2004)), and that forced overexpression of Nanog in ES cells promotes pluripotency when fused with neural stem cells (Silva et al., Nature, 441:997-1001 (2006)).

### Culture-Induced Reprogramming

Pluripotent cells have been derived from embryonic sources such as blastomeres and the inner cell mass (ICM) of the blastocyst (ES cells), the epiblast (EpiSC cells), primordial germ cells (EG cells), and postnatal spermatogonial stem cells ("maGSCsm" "ES-like" cells). Cells isolated from embryonic stem cells and germ cells are described for reference only. The following pluripotent cells, along with their donor cell/tissue is as follows: parthogenetic ES cells are derived from murine oocytes (Narasimha et al., Curr Biol, 7:881-884 (1997)); embryonic stem cells have been derived from blastomeres (Wakayama et al., Stem Cells, 25:986-993 (2007)); inner cell mass cells (source not applicable) (Eggan et al., Nature, 428:44-49 (2004)); embryonic germ and embryonal carcinoma cells have been derived from primordial germ cells (Matsui et al., Cell, 70:841-847 (1992)); GMCS, maSSC, and MASC have been derived from spermatogonial stem cells (Guan et al., Nature, 440:1199-1203 (2006); Kanatsu-Shinohara et al., Cell, 119:1001-1012 (2004); and Seandel et al., Nature, 449:346-350 (2007)); EpiSC cells are derived from epiblasts (Brons et al., Nature, 448:191-195 (2007); Tesar et al., Nature, 448:196-199(2007)); parthogenetic ES cells have been derived from human oocytes (Cibelli et al., Science, 295L819 (2002); Revazova et al., Cloning Stem Cells, 9:432-449 (2007)); human ES cells have been derived from human blastocysts (Thomson et al., Science, 282:1145-1147 (1998)); MAPC have been derived from bone marrow (Jiang et al., Nature, 418:41-49 (2002); Phinney and Prockop, Stem Cells, 25:2896-2902 (2007)); cord blood cells (derived from cord blood) (van de Ven et al., Exp Hematol, 35:1753-1765 (2007)); neurosphere derived cells derived from neural cell (Clarke et al., Science, 288:1660-1663 (2000)). Donor cells from the germ cell lineage such as PGCs or spermatogonial stem cells are known to be unipotent *in vivo*, but it has been shown that pluripotent ES-like cells (Kanatsu-Shinohara et al., Cell, 119:1001-1012 (2004) or maGSCs (Guan et al., Nature, 440:1199-1203 (2006), can be isolated after prolonged *in vitro* culture. While most of these pluripotent cell types were capable of in vitro differentiation and teratoma formation, only ES, EG, EC, and the spermatogonial stem cell-derived maGCSs or ES-like cells were pluripotent by more stringent criteria, as they were able to form postnatal chimeras and contribute to the germline. Recently, multipotent adult spermatogonial stem cells (MASCs) were derived from testicular spermatogonial stem cells of adult mice, and these cells had an expression profile different from that of ES cells (Seandel et al., Nature, 449:346-350 (2007)) but similar to EpiSC cells, which were derived from the epiblast of postimplantation mouse embryos (Brons et al., Nature, 448:191-195 (2007); Tesar et al., Nature, 448:196-199 (2007)).

### Reprogramming by Defined Transcription Factors

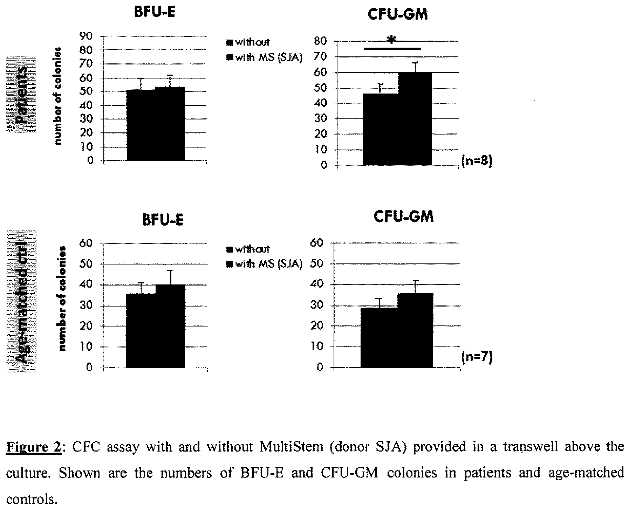

Takahashi and Yamanaka have reported reprogramming somatic cells back to an ES-like state (Takahashi and Yamanaka, Cell, 126:663-676 (2006)). They successfully reprogrammed mouse embryonic fibroblasts (MEFs) and adult fibroblasts to pluripotent ES-like cells after viral-mediated transduction of the four transcription factors Oct4, Sox2, c-myc, and Klf4 followed by selection for activation of the Oct4 target gene Fbx15 (Figure 2A). Cells that had activated Fbx15 were coined iPS (induced pluripotent stem) cells and were shown to be pluripotent by their ability to form teratomas, although they were unable to generate live chimeras. This pluripotent state was dependent on the continuous viral expression of the transduced Oct4 and Sox2 genes, whereas the endogenous Oct4 and Nanog genes were either not expressed or were expressed at a lower level than in ES cells, and their respective promoters were found to be largely methylated. This is consistent with the conclusion that the Fbx15-iPS cells did not correspond to ES cells but may have represented an incomplete state of reprogramming. While genetic experiments had established that Oct4 and Sox2 are essential for pluripotency (Chambers and Smith, Oncogene, 23:7150-7160 (2004); Ivanona et al., Nature, 442:5330538 (2006); Masui et al., Nat Cell Biol, 9:625-635 (2007)), the role of the two oncogenes c-myc and Klf4 in reprogramming is less clear. Some of these oncogenes may, in fact, be dispensable for reprogramming, as both mouse and human iPS cells have been obtained in the absence of c-myc transduction, although with low efficacy (Nakagawa et al., Nat Biotechnol, 26:191-106 (2008); Werning et al., Nature, 448:318-324 (2008); Yu et al., Science, 318: 1917-1920 (2007)).

### MAPC

Human MAPCs are described in U.S. Patent 7,015,037. MAPCs have been identified in other mammals. Murine MAPCs, for example, are also described in U.S. Patent 7,015,037. Rat MAPCs are also described in U.S. Patent No. 7,838,289.

These references describe MAPCs first isolated by Catherine Verfaillie.

### Isolation and Growth of MAPCs

Methods of MAPC isolation are known in the art. See, for example, U.S. Patent 7,015,037, and these methods, along with the characterization (phenotype) of MAPCs. MAPCs can be isolated from multiple sources, including, but not limited to, bone marrow, placenta, umbilical cord and cord blood, muscle, brain, liver, spinal cord, blood or skin. It is, therefore, possible to obtain bone marrow aspirates, brain or liver biopsies, and other organs, and isolate the cells using positive or negative selection techniques available to those of skill in the art, relying upon the genes that are expressed (or not expressed) in these cells (e.g., by functional or morphological assays such as those disclosed in the above-referenced applications).

MAPCs have also been obtained by modified methods described in Breyer et al., Experimental Hematology, 34:1596-1601 (2006) and Subramanian et al., Cellular Programming and Reprogramming: Methods and Protocols; S. Ding (ed.), Methods in Molecular Biology, 636:55-78 (2010).

### MAPCs from Human Bone Marrow as Described in U.S. Patent 7,015,037

MAPCs do not express the common leukocyte antigen CD45 or erythroblast specific glycophorin-A (Gly-A). The mixed population of cells was subjected to a Ficoll Hypaque separation. The cells were then subjected to negative selection using anti-CD45 and anti-Gly-A antibodies, depleting the population of CD45⁺ and Gly-A⁺ cells, and the remaining approximately 0.1% of marrow mononuclear cells were then recovered. Cells could also be plated in fibronectin-coated wells and cultured as described below for 2-4 weeks to deplete the cells of CD45⁺ and Gly-A⁺ cells. In cultures of adherent bone marrow cells, many adherent stromal cells undergo replicative senescence around cell doubling 30 and a more homogenous population of cells continues to expand and maintains long telomeres.

Alternatively, positive selection could be used to isolate cells via a combination of cell-specific markers. Both positive and negative selection techniques are available to those of skill in the art, and numerous monoclonal and polyclonal antibodies suitable for negative selection purposes are also available in the art (see, for example, Leukocyte Typing V, Schlossman, et al., Eds. (1995) Oxford University Press) and are commercially available from a number of sources.

Techniques for mammalian cell separation from a mixture of cell populations have also been described by Schwartz, et al., in U. S. Patent No. 5,759,793 (magnetic separation), Basch et al., 1983 (immunoaffinity chromatography), and Wysocki and Sato, 1978 (fluorescence-activated cell sorting).

Cells may be cultured in low-serum or serum-free culture medium. Serum-free medium used to culture MAPCs is described in U.S. Patent 7,015,037. Commonly-used growth factors include but are not limited to platelet-derived growth factor and epidermal growth factor. See, for example, U.S. Patent Nos. 7,169,610; 7,109,032; 7,037,721; 6,617,161; 6,617,159; 6,372,210;6,224,860; 6,037,174; 5,908,782; 5,766,951; 5,397,706; and 4,657,866; all teach growing cells in serum-free medium.

### Additional Culture Methods

In additional experiments the density at which MAPCs are cultured can vary from about 100 cells/cm² or about 150 cells/cm² to about 10,000 cells/cm², including about 200 cells/cm² to about 1500 cells/cm² to about 2000 cells/cm². The density can vary between species. Additionally, optimal density can vary depending on culture conditions and source of cells. It is within the skill of the ordinary artisan to determine the optimal density for a given set of culture conditions and cells.

Also, effective atmospheric oxygen concentrations of less than about 10%, including about 1-5% and, especially, 3-5%, can be used at any time during the isolation, growth and differentiation of MAPCs in culture.

Cells may be cultured under various serum concentrations, e.g., about 2-20%. Fetal bovine serum may be used. Higher serum may be used in combination with lower oxygen tensions, for example, about 15-20%. Cells need not be selected prior to adherence to culture dishes. For example, after a Ficoll gradient, cells can be directly plated, e.g., 250,000-500,000/cm². Adherent colonies can be picked, possibly pooled, and expanded.

In one embodiment, used in the experimental procedures in the Examples, high serum (around 15-20%) and low oxygen (around 3-5%) conditions were used for the cell culture. Specifically, adherent cells from colonies were plated and passaged at densities of about 1700-2300 cells/cm² in 18% serum and 3% oxygen (with PDGF and EGF).

In an embodiment specific for MAPCs, supplements are cellular factors or components that allow MAPCs to retain the ability to differentiate into cell types of more than one embryonic lineage, such as all three lineages. This may be indicated by the expression of specific markers of the undifferentiated state, such as Oct 3/4 (Oct 3A) and/or markers of high expansion capacity, such as telomerase.

### Cell Culture

For all the components listed below, see U.S. 7,015,037.

In general, cells useful for the invention can be maintained and expanded in culture medium that is available and well-known in the art. Also contemplated is supplementation of cell culture medium with mammalian sera. Additional supplements can also be used advantageously to supply the cells with the necessary trace elements for optimal growth and expansion. Hormones can also be advantageously used in cell culture. Lipids and lipid carriers can also be used to supplement cell culture media, depending on the type of cell and the fate of the differentiated cell. Also contemplated is the use of feeder cell layers.

Cells in culture can be maintained either in suspension or attached to a solid support, such as extracellular matrix components. Stem cells often require additional factors that encourage their attachment to a solid support, such as type I and type II collagen, chondroitin sulfate, fibronectin, "superfibronectin" and fibronectin-like polymers, gelatin, poly-D and poly-L-lysine, thrombospondin and vitronectin. The present disclosure may utilize fibronectin. See, for example, Ohashi et al., Nature Medicine, 13:880-885 (2007); Matsumoto et al., J Bioscience and Bioengineering, 105:350-354 (2008); Kirouac et al., Cell Stem Cell, 3:369-381 (2008); Chua et al., Biomaterials, 26:2537-2547 (2005); Drobinskaya et al., Stem Cells, 26:2245-2256 (2008); Dvir-Ginzberg et al., FASEB J, 22:1440-1449 (2008); Turner et al., J Biomed Mater Res Part B: Appl Biomater, 82B:156-168 (2007); and Miyazawa et al., Journal of Gastroenterology and Hepatology, 22:1959-1964 (2007)).

Cells may also be grown in "3D" (aggregated) cultures. An example is PCT/US2009/31528, filed January 21, 2009.

Once established in culture, cells can be used fresh or frozen and stored as frozen stocks, using, for example, DMEM with 40% FCS and 10% DMSO. Other methods for preparing frozen stocks for cultured cells are also available to those of skill in the art.

### Pharmaceutical Formulations

U.S. 7,015,037 teaches pharmaceutical formulations. The cell populations may be present within a composition adapted for and suitable for delivery, i.e., physiologically compatible.

The purity of the cells (or conditioned medium) for administration to a subject may be about 100% (substantially homogeneous). It may be 95% to 100%. It may be 85% to 95%. Particularly, in the case of admixtures with other cells, the percentage can be about 10%-15%, 15%-20%, 20%-25%, 25%-30%, 30%-35%, 35%-40%, 40%-45%, 45%-50%, 60%-70%, 70%-80%, 80%-90%, or 90%-95%. Or isolation/purity can be expressed in terms of cell doublings where the cells have undergone, for example, 10-20, 20-30, 30-40, 40-50 or more cell doublings.

The choice of formulation for administering the cells for a given application will depend on a variety of factors. Prominent among these will be the species of subject, the nature of the condition being treated, its state and distribution in the subject, the nature of other therapies and agents that are being administered, the optimum route for administration, survivability via the route, the dosing regimen, and other factors that will be apparent to those skilled in the art. For instance, the choice of suitable carriers and other additives will depend on the exact route of administration and the nature of the particular dosage form.

Final formulations of the aqueous suspension of cells/medium will typically involve adjusting the ionic strength of the suspension to isotonicity (i.e., about 0.1 to 0.2) and to physiological pH (i.e., about pH 6.8 to 7.5). The final formulation will also typically contain a fluid lubricant.

The cells/medium may be formulated in a unit dosage injectable form, such as a solution, suspension, or emulsion. Pharmaceutical formulations suitable for injection of cells/medium typically are sterile aqueous solutions and dispersions. Carriers for injectable formulations can be a solvent or dispersing medium containing, for example, water, saline, phosphate buffered saline, polyol (for example, glycerol, propylene glycol, liquid polyethylene glycol, and the like), and suitable mixtures thereof.

The skilled artisan can readily determine the amount of cells and optional additives, vehicles, and/or carrier in compositions to be administered. Typically, any additives (in addition to the cells) are present in an amount of 0.001 to 50 wt % in solution, such as in phosphate buffered saline. The active ingredient is present in the order of micrograms to milligrams, such as about 0.0001 to about 5 wt %, preferably about 0.0001 to about 1 wt %, most preferably about 0.0001 to about 0.05 wt % or about 0.001 to about 20 wt %, preferably about 0.01 to about 10 wt %, and most preferably about 0.05 to about 5 wt %.

The cells may be encapsulated for administration, particularly where encapsulation enhances the effectiveness of the therapy, or provides advantages in handling and/or shelf life. Cells may be encapsulated by membranes, as well as capsules, prior to implantation. It is contemplated that any of the many methods of cell encapsulation available may be employed.

A wide variety of materials may be used for microencapsulation of cells. Such materials include, for example, polymer capsules, alginate-poly-L-lysine-alginate microcapsules, barium poly-L-lysine alginate capsules, barium alginate capsules, polyacrylonitrile/polyvinylchloride (PAN/PVC) hollow fibers, and polyethersulfone (PES) hollow fibers.

Techniques for microencapsulation of cells that may be used for administration of cells are known to those of skill in the art and are described, for example, in Chang, P., et al., 1999; Matthew, H.W., et al., 1991; Yanagi, K., et al., 1989; Cai Z.H., et al., 1988; Chang, T.M., 1992 and in U.S. Patent No. 5,639,275 (which, for example, describes a biocompatible capsule for long-term maintenance of cells that stably express biologically active molecules. Additional methods of encapsulation are in European Patent Publication No. 301,777 and U.S. Pat. Nos. 4,353,888; 4,744,933; 4,749,620; 4,814,274; 5,084,350; 5,089,272; 5,578,442; 5,639,275; and 5,676,943.

The cells may be incorporated into a polymer, such as a biopolymer or synthetic polymer. Examples of biopolymers include, but are not limited to, fibronectin, fibrin, fibrinogen, thrombin, collagen, and proteoglycans. Other factors, such as the cytokines discussed above, can also be incorporated into the polymer. Cells may be incorporated in the interstices of a three-dimensional gel. A large polymer or gel, typically, will be surgically implanted. A polymer or gel that can be formulated in small enough particles or fibers can be administered by other common, more convenient, non-surgical routes.

The dosage of the cells will vary within wide limits and will be fitted to the individual requirements in each particular case. In general, in the case of parenteral administration, it is customary to administer from about 0.01 to about 20 million cells/kg of recipient body weight. The number of cells will vary depending on the weight and condition of the recipient, the number or frequency of administrations, and other variables known to those of skill in the art. The cells can be administered by a route that is suitable for the tissue or organ. For example, they can be administered systemically, i.e., parenterally, by intravenous administration, or can be targeted to a particular tissue or organ; they can be administrated via subcutaneous administration or by administration into specific desired tissues.

The cells can be suspended in an appropriate excipient in a concentration from about 0.01 to about 5x10⁶ cells/ml. Suitable excipients for injection solutions are those that are biologically and physiologically compatible with the cells and with the recipient, such as buffered saline solution or other suitable excipients. The composition for administration can be formulated, produced, and stored according to standard methods complying with proper sterility and stability.

### Administration into Lymphohematopoietic Tissues

Techniques for administration into these tissues are known in the art. For example, intra-bone marrow injections can involve injecting cells directly into the bone marrow cavity typically of the posterior iliac crest but may include other sites in the iliac crest, femur, tibia, humerus, or ulna; splenic injections could involve radiographic guided injections into the spleen or surgical exposure of the spleen via laparoscopic or laparotomy; Peyer's patches, GALT, or BALT injections could require laparotomy or laparoscopic injection procedures.

### Dosing

Doses for humans or other mammals can be determined without undue experimentation by the skilled artisan, from this disclosure, the documents cited herein, and the knowledge in the art. The dose of cells/medium appropriate to be used in accordance with the second aspect of the invention will depend on numerous factors. The parameters that will determine optimal doses to be administered for primary and adjunctive therapy generally will include some or all of the following: the disease being treated and its stage; the species of the subject, their health, gender, age, weight, and metabolic rate; the subject's immunocompetence; other therapies being administered; and expected potential complications from the subject's history or genotype. The parameters may also include: whether the cells are syngeneic, autologous, allogeneic, or xenogeneic; their potency (specific activity); the site and/or distribution that must be targeted for the cells/medium to be effective; and such characteristics of the site such as accessibility to cells/medium and/or engraftment of cells. Additional parameters include co-administration with other factors (such as growth factors and cytokines). The optimal dose in a given situation also will take into consideration the way in which the cells/medium are formulated, the way they are administered, and the degree to which the cells/medium will be localized at the target sites following administration.

The optimal dose of cells could be in the range of doses used for autologous, mononuclear bone marrow transplantation. For fairly pure preparations of cells, optimal doses may range from 10⁴ to 10⁸ cells/kg of recipient mass per administration. The optimal dose per administration may be between 10⁵ to 10⁷ cells/kg. The optimal dose per administration may be 5x10⁵ to 5x10⁶ cells/kg. By way of reference, higher doses in the foregoing are analogous to the doses of nucleated cells used in autologous mononuclear bone marrow transplantation. Some of the lower doses are analogous to the number of CD34⁺ cells/kg used in autologous mononuclear bone marrow transplantation.

The cells/medium may be administered in an initial dose, and thereafter maintained by further administration. Cells/medium may be administered by one method initially, and thereafter administered by the same method or one or more different methods. The levels can be maintained by the ongoing administration of the cells/medium. The cells/medium may be administered either initially or to maintain their level in the subject or both by intravenous injection. Other forms of administration may be used, dependent upon the patient's condition and other factors, discussed elsewhere herein.

Cells/medium may be administered in many frequencies over a wide range of times. Generally lengths of treatment will be proportional to the length of the disease process, the effectiveness of the therapies being applied, and the condition and response of the subject being treated.

Because the invention provides cells for use in a method for treating MDS, this could potentially be extended to other immune/environmental bone marrow failure states, such as, aplastic anemia, immune-mediated chronic neutropenia, and large granular lymphocytic leukemia. The underlying idea is that increasing proliferation and/or differentiation or reducing apoptosis of myeloid precursors, while effective for MDS, provides a principle by which other myeloid cell deficiencies could be reduced or even eliminated.

### Uses

Administering the cells is useful to reduce any of the overt symptoms of MDS as described in this application. This may be based on underlying effects of the cells, such as, reduction in the decline of myeloid precursors and/or mature myeloid blood cells, as described elsewhere in this application.

In addition, other uses are provided by knowledge of the biological mechanisms described in this application. One of these includes drug discovery. This aspect involves screening one or more compounds for the ability to affect the cell's ability to achieve any of the effects described in this application. Accordingly, the assay may be designed to be conducted *in vivo* or *in vitro.* Assays could assess the effect at any desired level, e.g., morphological, e.g., hematological, immunological survival, including, effect on apoptosis and effect on differentiation of blood cells/myeloid precursors.

The cells may be screened for an agent that enhances the cells' ability to prevent or reduce the events associated with MDS as described in this application. Assessment could be *in vivo* as in appropriate animal models.

Without intending to be found by any particular theory, the effects of the cells may be occurring by direct effects on one or more of the various myeloid blood cells and their progenitors or by the secretion of factors from these cells that act on these progenitors to increase proliferation and/or differentiation. Accordingly, the assays for potency described in this application can include *in vitro* assays that measure the effects of the cells on any of the various myeloid cells and their progenitors. This would include assays on general viability, including, proliferation, lifespan, and differentiation. It may also involve analysis of the function of those hematopoietic cells, including, appropriate or inappropriate gene expression. Such assays include the colony forming assays shown in the Examples.

Gene expression can be assessed by directly assaying protein or RNA. This can be done through any of the well-known techniques available in the art, such as by FACS and other antibody-based detection methods and PCR and other hybridization-based detection methods. Indirect assays may also be used for expression, such as the effect of gene expression.

Assays for potency may be performed by detecting genes that are modulated by the cells or by detecting genes that are expressed by the cells and which may be responsible for the ameliorative effects described in this application, for example, osteopontin, stem cell factor, and Angpt1, which are hematopoietic stem cell maintenance genes and, therefore, are of interest. Detection may be direct, e.g., via RNA or protein assays or indirect, e.g., biological assays for one or more biological effects of these genes.

Assays for expression/secretion include, but are not limited to, ELISA, Luminex. qRT-PCR, anti-factor western blots, and factor immunohistochemistry on tissue samples or cells.

Quantitative determination of modulatory factors in cells and conditioned media can be performed using commercially available assay kits (e.g., R&D Systems that relies on a two-step subtractive antibody-based assay).

The cells described herein can be used as a feeder layer to maintain hematopoiesis of bone marrow mononuclear cells in MDS patients.

A further use for the method of the invention is the establishment of cell banks to provide cells for clinical administration. Generally, a fundamental part of this procedure is to provide cells that have a desired potency for administration in various therapeutic clinical settings.

The cells may be selected for having a desired potency for enhancing mature myeloid blood cell levels *in vivo* or an *in vitro* context. Medium conditioned by the cells or to extracts of such conditioned medium can also be used to assess potency of a cellular preparation, e.g., by increasing differentiation and/or proliferation, or reducing apoptosis of one or more of the myeloid precursor cells, such as those shown in Figure 6, and/or increasing the number of mature myeloid cells.

Any of the same assays useful for drug discovery could also be applied to selecting cells for the bank as well as from the bank for administration.

Accordingly, in a banking procedure, the cells (or medium) would be assayed for the ability to achieve any of the above effects. Then, cells would be selected that have a desired potency for any of the above effects, and these cells would form the basis for creating a cell bank.

It is also contemplated that potency can be increased by treatment with an exogenous compound, such as a compound discovered through screening the cells with large combinatorial libraries. These compound libraries may be libraries of agents that include, but are not limited to, small organic molecules, antisense nucleic acids, siRNA DNA aptamers, peptides, antibodies, non-antibody proteins, cytokines, chemokines, and chemo-attractants. For example, cells may be exposed to such agents at any time during the growth and manufacturing procedure. The only requirement is that there be sufficient numbers for the desired assay to be conducted to assess whether or not the agent increases potency. Such an agent, found during the general drug discovery process described above, could more advantageously be applied during the last passage prior to banking.

One embodiment that has been applied successfully to MultiStem® is as follows. Cells can be isolated from a qualified marrow donor that has undergone specific testing requirements to determine that a cell product that is obtained from this donor would be safe to be used in a clinical setting. The mononuclear cells are isolated using either a manual or automated procedure. These mononuclear cells are placed in culture allowing the cells to adhere to the treated surface of a cell culture vessel. The MultiStem® cells are allowed to expand on the treated surface with media changes occurring on day 2 and day 4. On day 6, the cells are removed from the treated substrate by either mechanical or enzymatic means and replated onto another treated surface of a cell culture vessel. On days 8 and 10, the cells are removed from the treated surface as before and replated. On day 13, the cells are removed from the treated surface, washed and combined with a cryoprotectant material and frozen, ultimately, in liquid nitrogen. After the cells have been frozen for at least one week, an aliquot of the cells is removed and tested for potency, identity, sterility and other tests to determine the usefulness of the cell bank. These cells in this bank can then be used by thawing them, placing them in culture or use them out of the freeze to treat potential indications.

Another use is a diagnostic assay for efficacy and beneficial clinical effect following administration of the cells. Depending on the indication, there may be biomarkers available to assess. The dosage of the cells can be adjusted during the treatment according to the effect.

The diagnostic assay may involve assessing the hematopoietic colony forming capacity by using CFC and/or LTC-IC assays.

A further use is to assess the efficacy of the cell to achieve any of the above results as a pre-treatment diagnostic that precedes administering the cells to a subject. Moreover, dosage can depend upon the potency of the cells that are being administered. Accordingly, a pre-treatment diagnostic assay for potency can be useful to determine the dose of the cells initially administered to the patient and, possibly, further administered during treatment based on the real-time assessment of clinical effect.

The pre-treatment diagnostic procedure may involve assessing the potency of the cells to enhance formation of myeloid colonies in a CFC and/or LTC-IC assay.

It is also to be understood that the cells described herein can be used not only for purposes of treatment, but also research purposes, both *in vivo* and *in vitro* to understand the mechanism involved normally and in disease models. Assays, *in vivo* or *in vitro,* can be done in the presence of agents known to be involved in the biological process. The effect of those agents can then be assessed. These types of assays could also be used to screen for agents that have an effect on the events that are promoted by the cells described herein. Accordingly, one could screen for agents in the disease model that reverse the negative effects and/or promote positive effects. Conversely, one could screen for agents that have negative effects in a non-disease model.

The source of the cells for the various assays could be blood and/or bone marrow from normal as well as MDS subjects.

### Compositions

Also described herein are cell populations with specific potencies for achieving any of the effects described herein. As described above, these populations are established by selecting for cells that have desired potency. These populations are used to make other compositions, for example, a cell bank comprising populations with specific desired potencies and pharmaceutical compositions containing a cell population with a specific desired potency.

The cells may have a desired potency to increase mature blood cell levels, e.g., red blood cells, leukocytes, and platelets.

### NON-LIMITING EXAMPLES

### Example 1

### 1. The effect of human MultiStem/MAPC on hematopoietic stem cells (HSCs) derived from MDS patients using colony formation assays (short- and long-term cultures).

### 1.1 Characteristics of patient samples

Bone marrow (BM) was obtained from presumed MDS patients and healthy controls, after informed consent. All sampling and handling were conducted in accordance with the guidelines of the local ethical committee of the University Hospitals Leuven (UZ Leuven), which comply with the Helsinki declaration.

So far, twenty-four BM samples from presumed MDS patients were analyzed. After clinical diagnosis, thirteen patients were classified as low-risk MDS and four patients as high-risk MDS. The other patients were categorized as unknown cytopenias (n=7). As controls, we used young (n=16) and age-matched (n=7) BM samples from healthy volunteers.

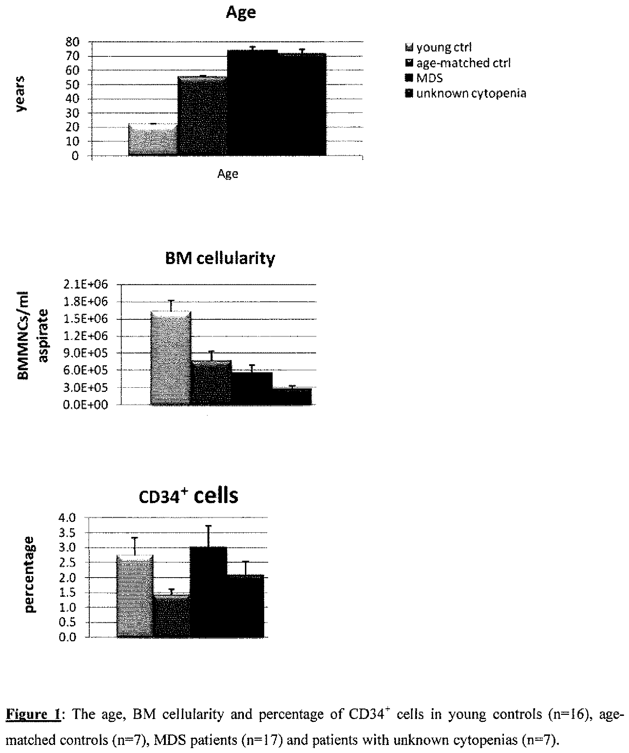

BM mononuclear cells (BMMNCs) were isolated over a Ficoll-Hypaque gradient (Sigma) and CD34⁺ cells selected using magnetic beads (Miltenyi Biotec). The cellularity of the BM decreases with age, which was observed in patients as well as age-matched controls. However, the percentage of CD34⁺ cells was not affected in patients with MDS or unknown cytopenias. (Figure 1)

### 1.2 Colony forming cell (CFC) assay

BMMNCs were prepared in MethoCult H4434 media containing 30% FBS, 50ng/ml stem cell factor, 10ng/ml GM-CSF, 10ng/ml interleukin-3 and 3U/ml EPO (Stem Cell Technologies). Cells were plated at 1.5x10⁵ cells/well for patients and 1.5x10⁴cells/well for controls in a 12-well plate, with or without MultiStem/MAPC. In the condition with MultiStem, cells were added at 10.000 cells/cm² in a 0.4µm transwell above the culture (= non-contact). Following 14 days of incubation, plates were scored using an inverted microscope. Groups of 40 or more cells were scored as a colony. Granulocyte, monocyte and granulocyte-monocyte colonies were scored as CFU-GM and erythroid colonies as BFU-E.

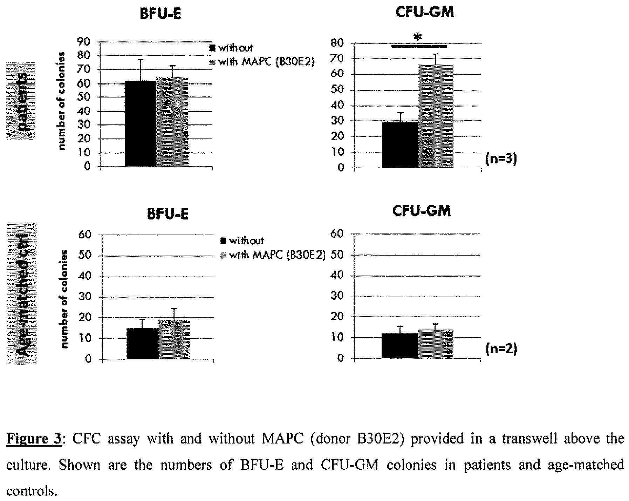

There was no significant increase in the number of erythroid colonies when MultiStem or MAPC were added to the culture, both in patients and age-matched controls. However, a significant increase in myeloid colonies (CFU-GM) was seen in the patients treated with MultiStem or MAPC (Figure 2-3).

### 1.3 Long-term culture initiating cell (LTC-IC) assay

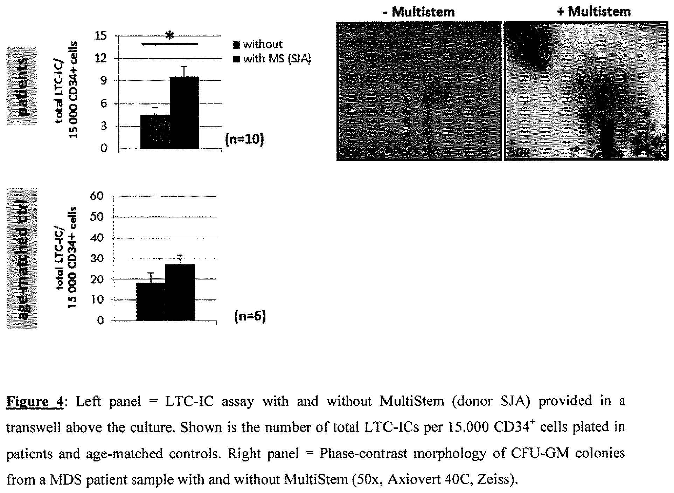

To determine the influence of MultiStem/MAPC on the frequency of primitive hematopoietic progenitor cells, a LTC-IC assay was initiated with and without MultiStem provided in a 0.4µm transwell above the culture (10.000 cells/cm²). Therefore, CD34⁺ cells derived from MDS patients and controls were plated on AFT feeders for 5 weeks. Subsequently, cells were replated in methylcellulose and colonies were quantified after 14 days. In MDS patient samples, a significantly higher frequency of LTC-ICs was observed when MultiStem was added to the culture, whereas no influence was seen on the frequency of LTC-ICs in age-matched control samples (Figure 4).

Next, the same assay was repeated in patients with low-risk MDS and unknown cytopenias (n=5), including additional conditions: a non-contact culture with two different MultiStem donors (SJA and SVG), a non-contact culture with fresh MultiStem medium (without any cells), a direct contact culture with MultiStem (donor SJA, plated at 5.000 cells/cm²) and finally a non-contact culture with MultiStem (donor SJA) provided intermittently (every other week).

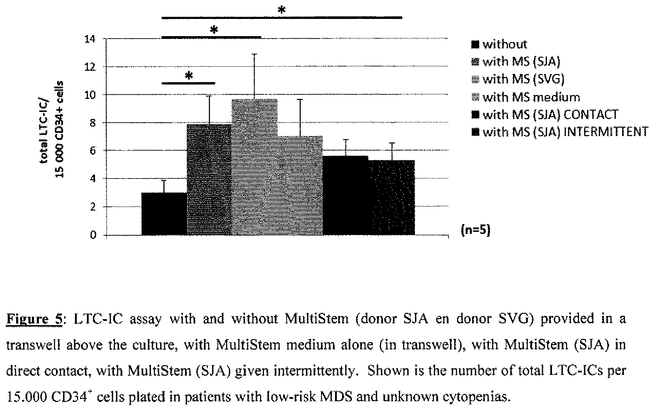

We could confirm the previous result in which MultiStem (donor SJA) increases significantly the percentage of LTG-ICs. Moreover, this effect was donor-independent, as the same result could be obtained using MultiStem donor SVG. The positive effect of MultiStem was also maintained when the cells were provided intermittently (every other week) to the culture. No significant result was obtained with MultiStem medium alone or when MultiStem was in direct contact with the culture, indicating a possible role for a soluble factor produced by MultiStem (Figure 5).

### Example 2

### 1. The effect of Multipotent Adult Progenitor Cells on bone marrow failure in Myelodysplastic Syndromes

### Introduction

Primary myelodysplastic syndromes (MDS) are clonal hematopoietic stem cell (HSC) disorders characterized by ineffective hematopoiesis and peripheral cytopenias. Intrinsic defects in the HSC as well as extrinsic defects in the bone marrow (BM) niche all contribute to the MDS pathogenesis. In some patients, immunomodulatory drugs have shown a significant improvement in cytopenias. Multipotent Adult Progenitor Cells (MAPC) are non-hematopoietic stromal stem cells derived from BM with potent immunomodulatory effects towards T cells.

### Purpose

Test the effect of MAPC as a cell-based therapy for MDS. We hypothesize that low-risk MDS patients in whom immune- and environmental-mediated mechanisms are in large part causative for the cytopenias could benefit from MAPC therapy.

### Materials and Methods

Two MDS mouse models were generated: a first model was created by overexpressing the oncogene Evi-1 in-lin cells of murine BM that were subsequently transplanted into lethally irradiated C57BI/6 mice. A second model was created by intercrossing flexed Dicer with Osterix-Cre mice to create Osx- Cre⁺dicer^{lox/lox} (OCD) mice, in which Dicer is selectively deleted in BM osteoprogenitors. This deletion disrupts the integrity of hematopoiesis in the niche and leads to MDS.

Furthermore,BM samples from MDS patients were used in hematopoietic short- and long term cultures, with or without human MAPC seeded in transwells above the culture.

### Results

In the Evi-1 transplanted mice, pancytopenia developed 10-15 months after BM transplantation and Evi-1 was highly expressed in blood and BM. Moreover, the number of hematopoietic colonies and frequency of primitive progenitors was decreased in these mice. The OCD model showed lower blood counts, a decreased number of mixed colonies and lower frequency of hematopoietic progenitors as compared to control mice. Morphological analysis revealed dysplastic megakaryocytes in BM and increased polychromatophilic RBCs in blood of diseased mice.

Total BM cells, derived from MDS patients, were plated in methylcellulose with and without human MAPC. After 14 days, an increase in CFU-GM colonies was seen in the condition with MAPC. When CD34⁺ cells from these patients were plated on feeders in a long-term culture, we could observe a higher frequency of primitive hematopoietic progenitors when MAPC were provided in atranswell above the culture.

### Conclusions

We established two mouse models of MDS. Furthermore, human MAPC exerted a positive effect *in vitro* on the colony forming capacity of hematopoietic cells derived from MDS patients.

## Claims

1. An *in vitro* method to assess the potency of a cell, the cell being a multipotent adult progenitor cell **characterised in that** the cell is a non-embryonic, non-germ cell that expresses one or more of oct4, telomerase, rex-1, or rox-1, the method comprising assaying for the potency of the cell to enhance differentiation, proliferation, or lifespan (viability) of red blood cells, leukocytes, platelets, or precursors of these cells.

2. The method of claim 1 wherein the non-embryonic, non-germ cells express telomerase.

3. The method of claim 1 wherein the non-embryonic, non-germ cells can differentiate into cell types of at least two of endodermal, ectodermal, and mesodermal germ layers.

4. The method of claim 1 wherein the non-embryonic, non-germ cells express oct4.

5. The method of claim 3 wherein the non-embryonic, non-germ cells can differentiate into cell types of all three of the endodermal, ectodermal, and mesodermal germ layers.

6. The method of claim 2 wherein the non-embryonic, non-germ cells can differentiate into cell types of at least two of the endodermal, ectodermal, and mesodermal germ layers.

7. The method of claim 4 wherein the non-embryonic, non-germ cells can differentiate into cell types of at least two of the endodermal, ectodermal, and mesodermal germ layers.

8. The method of claim 1 wherein the non-embryonic, non-germ cells express oct4 and telomerase and can differentiate into cell types of all three of the endodermal, ectodermal, and mesodermal germ layers.

9. The method of any preceding claim wherein the non-embryonic, non-germ cells are not tumorigenic or transformed and have a normal karyotype.

10. The method of any preceding claim wherein the assaying for the potency of the cell includes a colony forming assay or wherein the assaying for the potency of the cell is performed by detecting genes that are modulated by the cells or by detecting genes that are expressed by the cells.

11. The method of claim 10, wherein the genes are selected from the group consisting of osteopontin, stem cell factor and Angpt1.

12. The method of claim 10 or claim 11, wherein the genes that are expressed by the cells are detected by ELISA, Luminex. qRT-PCR, anti-factor western blots or factor immunohistochemistry.

13. The method of any preceding claim further comprising selecting cells having said potency and optionally further comprising creating a cell bank comprising the cells having said potency.

14. The method of any preceding claim wherein the non-embryonic, non-germ cells are derived from human bone marrow.

15. Cells for use in a method for treating a myelodysplastic syndrome in a subject, the cells being non-embryonic, non-germ cells that express one or more of oct4, telomerase, rex-1, or rox-1, wherein the cells have been assessed for potency according to the method of any one of claims 1 to 14 and wherein the cells have been found to have a potency to enhance differentiation, proliferation, or lifespan (viability) of red blood cells, leukocytes, platelets, or precursors of these cells.

## Patentansprüche

1. *In vitro*-Verfahren zum Beurteilen der Potenz einer Zelle, wobei die Zelle eine multipotente adulte Vorläuferzelle ist, **dadurch gekennzeichnet, dass** die Zelle eine nicht-embryonale Nicht-Keimzelle ist, die eines oder mehrere von Oct4, Telomerase, Rex-1 oder Rox-1 exprimiert, wobei das Verfahren das Testen auf die Potenz der Zelle, die Differenzierung, Proliferation oder Lebensdauer (Lebensfähigkeit) von roten Blutzellen, Leukozyten, Blutplättchen oder Vorläufern dieser Zellen zu verbessern, umfasst.

2. Verfahren nach Anspruch 1, wobei die nicht-embryonalen Nicht-Keimzellen Telomerase exprimieren.

3. Verfahren nach Anspruch 1, wobei die nicht-embryonalen Nicht-Keimzellen sich zu Zelltypen von mindestens zwei der endodermalen, ektodermalen und mesodermalen Keimschichten differenzieren können.

4. Verfahren nach Anspruch 1, wobei die nicht-embryonalen Nicht-Keimzellen Oct4 exprimieren.

5. Verfahren nach Anspruch 3, wobei die nicht-embryonalen Nicht-Keimzellen sich zu Zelltypen von allen drei der endodermalen, ektodermalen und mesodermalen Keimschichten differenzieren können.

6. Verfahren nach Anspruch 2, wobei die nicht-embryonalen Nicht-Keimzellen sich zu Zelltypen von mindestens zwei der endodermalen, ektodermalen und mesodermalen Keimschichten differenzieren können.

7. Verfahren nach Anspruch 4, wobei die nicht-embryonalen Nicht-Keimzellen sich zu Zelltypen von mindestens zwei der endodermalen, ektodermalen und mesodermalen Keimschichten differenzieren können.

8. Verfahren nach Anspruch 1, wobei die nicht-embryonalen Nicht-Keimzellen Oct4 und Telomerase exprimieren und sich zu Zelltypen von allen drei der endodermalen, ektodermalen und mesodermalen Keimschichten differenzieren können.

9. Verfahren nach einem der vorhergehenden Ansprüche, wobei die nicht-embryonalen Nicht-Keimzellen nicht krebserzeugend oder transformiert sind und einen normalen Karyotyp aufweisen.

10. Verfahren nach einem der vorhergehenden Ansprüche, wobei der Test auf die Potenz der Zelle einen Koloniebildungstest beinhaltet oder wobei der Test auf die Potenz der Zelle durchgeführt wird, indem Gene nachgewiesen werden, die durch die Zellen moduliert werden, oder indem Gene nachgewiesen werden, die durch die Zellen exprimiert werden.

11. Verfahren nach Anspruch 10, wobei die Gene ausgewählt sind aus der Gruppe, bestehend aus Osteopontin, Stammzellenfaktor und Angpt1.

12. Verfahren nach Anspruch 10 oder Anspruch 11, wobei die Gene, die durch die Zellen exprimiert werden, durch ELISA, Luminex. qRT-PCR, Anti-Faktor-Western-Blots oder Faktor-Immunhistochemie nachgewiesen werden.

13. Verfahren nach einem der vorhergehenden Ansprüche, ferner umfassend das Auswählen von Zellen, welche die Potenz aufweisen, und gegebenenfalls ferner umfassend das Erzeugen einer Zellbank, welche die Zellen umfasst, die diese Potenz aufweisen.

14. Verfahren nach einem der vorhergehenden Ansprüche, wobei die nicht-embryonalen Nicht-Keimzellen aus menschlichem Knochenmark abgeleitet werden.

15. Zellen zur Verwendung in einem Verfahren zur Behandlung eines myelodysplastischen Syndroms bei einem Patienten, wobei die Zellen nicht-embryonale Nicht-Keimzellen sind, die eines oder mehrere von Oct4, Telomerase, Rex-1 oder Rox-1 exprimieren, wobei die Zellen gemäß dem Verfahren nach einem der Ansprüche 1 bis 14 auf ihre Potenz beurteilt wurden und wobei für die Zellen gefunden wurde, dass sie eine Potenz zur Verbesserung der Differenzierung, Proliferation oder Lebensdauer (Lebensfähigkeit) von roten Blutzellen, Leukozyten, Blutplättchen oder Vorläufern dieser Zellen aufweisen.

## Revendications

1. Procédé *in vitro* pour évaluer la puissance d'une cellule, la cellule étant une cellule progénitrice adulte multipotente, **caractérisé en ce que** la cellule est une cellule non embryonnaire non germinale qui exprime l'une ou plusieurs parmi l'oct4, la télomérase, la rex-1 ou la rox-1, le procédé comprenant le dosage de la puissance de la cellule pour augmenter la différentiation, la prolifération ou la longévité (la viabilité) de globules rouges, de leucocytes, de plaquettes ou de précurseurs de ces cellules.

2. Procédé selon la revendication 1, les cellules non embryonnaires non germinales exprimant la télomérase.

3. Procédé selon la revendication 1, les cellules non embryonnaires non germinales pouvant se différencier en types cellulaires d'au moins deux des feuillets embryonnaires endodermes, ectodermes et mésodermes.

4. Procédé selon la revendication 1, les cellules non embryonnaires non germinales exprimant l'oct4.

5. Procédé selon la revendication 3, les cellules non embryonnaires non germinales pouvant se différencier en types cellulaires des trois feuillets embryonnaires endodermes, ectodermes et mésodermes.

6. Procédé selon la revendication 2, les cellules non embryonnaires non germinales pouvant se différencier en types cellulaires d'au moins deux des feuillets embryonnaires endodermes, ectodermes et mésodermes.

7. Procédé selon la revendication 4, les cellules non embryonnaires non germinales pouvant se différencier en types cellulaires d'au moins deux des feuillets embryonnaires endodermes, ectodermes et mésodermes.

8. Procédé selon la revendication 1, les cellules non embryonnaires non germinales exprimant l'oct4 et la télomérase et pouvant se différencier en types cellulaires des trois feuillets embryonnaires endodermes, ectodermes et mésodermes.

9. Procédé selon l'une quelconque des revendications précédentes, les cellules non embryonnaires non germinales n'étant pas tumorigènes ou transformées et possédant un caryotype normal.

10. Procédé selon l'une quelconque des revendications précédentes, le dosage pour la puissance de la cellule comportant un dosage de formation de colonie ou le dosage pour la puissance de la cellule étant effectué par la détection de gènes qui sont modulés par les cellules ou par la détection de gènes qui sont exprimés par les cellules.

11. Procédé selon la revendication 10, les gènes étant choisis dans le groupe constitué par l'ostéopontine, le facteur de cellules souches et l'Angpt1.

12. Procédé selon la revendication 10 ou 11, les gènes qui sont exprimés par les cellules étant détectés par ELISA, Luminex. qRT-PCR, transfert Western d'anti-facteur ou par immunohistochimie de facteur.

13. Procédé selon l'une quelconque des revendications précédentes comprenant la sélection de cellules possédant ladite puissance et éventuellement comprenant en outre la création d'une banque de cellules comprenant les cellules possédant ladite puissante.

14. Procédé selon l'une quelconque des revendications précédentes, les cellules non embryonnaires non germinales étant issues de moelle osseuse humaine.

15. Cellules pour une utilisation dans un procédé pour le traitement d'un syndrome myélodysplasique chez un sujet, les cellules étant des cellules non embryonnaires non germinales qui expriment l'une ou plusieurs parmi l'oct4, la télomérase, la rex-1 ou la rox-1, les cellules ayant été dosées pour leur puissance conformément au procédé selon l'une quelconque des revendications 1 à 14 et les cellules s'étant avéré posséder une puissance pour augmenter la différentiation, la prolifération ou la longévité (la viabilité) de globules rouges, de leucocytes, de plaquettes ou de précurseurs de ces cellules.
